# EUROPÄISCHE PATENTSCHRIFT

(11) **EP 1 029 505 B1**
(45) Veröffentlichungstag und Bekanntmachung des Hinweises auf die Patenterteilung: **25.05.2005**
(21) Anmeldenummer: 00100142.9
(22) Anmeldetag: 07.01.2000
(51) Int. Cl.: A61B 3/107, A61B 3/16

(54) **Einrichtung zum Ermitteln topographischer Eigenschaften der Cornea**
Device for determining topographic properties of the cornea
Appareil pour déterminer des propriétés topographiques de la cornée

(30) Priorität: 20.01.1999 US 234212
(43) Veröffentlichungstag der Anmeldung: 23.08.2000
(73) Patentinhaber: Kamillo Eisner-Stiftung, 6052 Hergiswil/NW (CH)
(72) Erfinder: Grabner, Günther, Prof. Dr. MD, 1010 Wien (AT); Eilmsteiner, Reinhard, 3250 Wieselburg (AT); Husinsky, Wolfgang, Dipl.-Ing. Dr., 1030 Wien (AT)
(74) Vertreter: Althoff, Gerhard

(56) Entgegenhaltungen:
- AT-B- 400 664
- US-A- 4 172 447
- US-A- 4 209 021
- US-A- 5 031 623
- US-A- 5 190 042
- US-A- 5 307 096

## Beschreibung

Die Erfindung bezieht sich auf eine Einrichtung zum Ermitteln topographischer Eigenschaften der Cornea an einem Auge, mit einem zum Erfassen fotographischer Abbildungen ausgebildeten und mit einem Projektionskonus versehenen Videokeratoskop, einer daran angeordneten Trägereinheit für eine hinsichtlich der Lage und Orientierung relativ zu der Cornea elektromotorisch verschiebbare Positioniereinheit mit daran angeordneter Indentationseinheit, welche eine in den Strahlengang des Projektionskonus ragende und zur Erreichung einer Indentation der Cornea zuführbare Sonde aufweist.

Aus der AT-B 400 664 ist zum Ermitteln topographischer Eigenschaften durch mechanische Indentationen eine an einem handelsüblichen Videokeratoskop angeordnete und zum Vermessen der äusseren Krümmung der Cornea ausgebildete Einrichtung bekannt, bei welcher eine Sonde mit einer im µm-Bereich liegenden fixen Indentationstiefe mit der Cornea in Eingriff bringbar ist. Zur Feststellung lokaler Veränderungen des Corneagewebes sowie zur Früherkennung von Hornhautleiden wird von der natürlichen (ungedellten) Krümmung der Cornea eine erste Abbildung und anschliessend von der indentierten (gedellten) Krümmung der Cornea eine zweite Abbildung aufgenommen. Die Abbildungen (Fotos) werden mathematisch voneinander subtrahiert, analysiert und als präoperative Entscheidungshilfe für operative Korrekturen von Refraktionsanomalien oder für Transplantationen der Hornhaut herangezogen, wobei derartige ophthalmologische Eingriffe unter dem Begriff "Refraktive Chirurgie" bekannt sind.

Für andere ophthalmologische Eingriffe sind weiterhin Einrichtungen zum Messen des intraokularen Drucks (IOP) in einem Auge bekannt, welche beispielsweise nach der Impressionstonometrie oder Applanationstonometrie arbeiten. Der intraokulare Druck kann als Entscheidungshilfe für eine unter dem Begriff "Glaukomchirurgie" bekannte Operation herangezogen werden.

In Verbindung mit der Impressionstonometrie ist aus der US-A 4,172,447 eine Einrichtung bekannt, bei welcher mittels einer Düse ein gasförmiges Medium in Form eines Staustrahls auf das zu untersuchende Auge gerichtet wird. Die dabei etwa punktförmig bewirkte Eindringtiefe der Cornea wird von einem mit einem Pulsnehmer wirkverbundenen Messwandler erfasst, in elektrische Signale und Daten umgewandelt und mittels elektronischer Geräte gespeichert und angezeigt, wobei anhand der Signale und Daten auf den intraokularen Druck (IOP) des jeweils untersuchten Auges rückgeschlossen werden kann.

In Verbindung mit der Applanationstonometrie ist aus der US-A 5,190,042 eine Einrichtung bekannt, bei welcher ein Messkörper in Form eines Prismas mit vorgegebener Kraft gegen das Auge gedrückt wird und von der dabei bewirkten Applanationsfläche abhängige Signale und Daten ermittelt werden, anhand welcher auf den intraokularen Druck (IOP) des jeweils untersuchten Auges rückgeschlossen werden kann.

Weiterhin ist zur Diagnostizierung von Hautflächen aus der US-A 4,209,021 ein Gerät bekannt, welches einen zweiarmig ausgebildeten und an einer horizontalen Achse schwenkbar gelagerten Hebel umfasst, welcher an dem oberen Hebelarm mit einer Kontaktsonde und an dem unteren Hebelarm einen mit einem Magnetsystem zusammenwirkenden elektrischen Spulenkörper aufweist. Dieses Gerät ist aufgrund der unkontrollierbaren Schwenkbewegung des Hebelarms für den ophthalmologischen Anwendungsbereich mit im µm-Bereich liegenden Bewegungen nicht geeignet.

Der Erfindung liegt die Aufgabe zugrunde, eine Einrichtung zu schaffen, mittels welcher es möglich wird die topographischen Eigenschaften einer Cornea vor einem chirurgischen Eingriff beziehungsweise nach einem chirurgischen Eingriff zur Beurteilung und Auswertung pathologischer Veränderungen der Cornea zu erfassen, wobei die Veränderungen als Entscheidungshilfe für etwaige Operationen, Transplantationen oder zur Früherkennung von Krankheiten der Cornea erforderlich sind.

Gelöst wird diese Aufgabe gemäss der Erfindung durch die im kennzeichnenden Teil des Anspruchs 1 angegebenen Merkmale.

Mit der erfindungsgemässen Einrichtung kann der Augenchirurg (Ophthalmologe) beispielsweise vor Beginn eines operativen Eingriffs, insbesondere vor Beginn eines refraktiven Eingriffs an der Cornea eines Auges eine exakte Analyse durchführen, mittels welcher beispielsweise die von der Dicke und Elastizität (E-Modul) sowie von lokalen Variationen abhängigen mechani-schen Eigenschaften der Cornea festgestellt und zur Bestimmung der Modalitäten für den Eingriff herangezogen werden.

Die kraftabhängigen Indentation der Cornea hat sich in Bezug auf die an sich bekannte und mit fixer Tiefe durchgeführte Indentation der Cornea als vorteilhaft herausgestellt. Mit der kraftabhängigen Indentation können nunmehr Vergleiche von Hornhautleiden und Hornhautanomalien verschiedener Patienten und Altersgruppen sowie Geschlechter durchgeführt und von den ermittelten Daten sogenannte Normkurven erstellt werden.

Die erfindungsgemässe Einrichtung gewährleistet eine sichere Funktion bei der Indentation, wobei ein besonderer Vorteil darin besteht, dass die Sonde mit dem zweiten Tragbügel unter Zwischenschaltung des Elektromagneten beim Überschreiten der vorgegebenen Indentationskraft selbsttätig und kontrolliert durch eine geringe Bewegung des zweiten Tragbügels relativ zu dem Elektromagneten ausser Eingriff der Vorderfläche der Cornea bringbar ist und somit eine Verletzung der Cornea verhindert wird.

Die mit geeigneten Mitteln aus der Analyse umgewandelten Daten beziehungsweise aus den Daten abgeleiteten Parameter dienen als Grundlage für eine Früherkennung bestimmter pathologischer und die Sehkraft beeinträchtigende Veränderungen der Cornea und können als präoperative und postoperative Grundlage für eine Analyse der Corneabeschaffenheit herangezogen werden. Mit der erfindungsgemässen Einrichtung besteht somit die Möglichkeit einer dynamischen Analyse der Cornea (**DCI** = Dynamic Corneal Imaging).

Ein weiterer Vorteil ergibt sich, wenn die Indentation sowie Erfassung davon abhängiger Daten bei dem jeweiligen Patienten pulssynchron durchgeführt wird und die Abbildungen immer in der gleichen Phase des Herzschlages erfasst werden. Hiermit werden etwaige Verfälschungen durch pulsbedingte Bewegungen des Auges weitgehend ausgeschlossen.

Weitere Vorteile, Merkmale und Einzelheiten der Erfindung ergeben sich aus der nachstehenden Beschreibung und der Zeichnung sowie den einzelnen Patentansprüchen.

Die Erfindung wird nachstehend in Verbindung mit einem in der Zeichnung dargestellten Ausführungsbeispiel der Einrichtung sowie die einzelnen Funktionsabläufe beschrieben. Es zeigt:
- **Fig.1**: eine in einer ersten räumlichen Seitenansicht dargestellte und mit einem optischen Vermessungsgerät sowie einer Positioniereinheit versehene Einrichtung zum Erfassen der Topologie der Vorderfläche einer Cornea;
- **Fig.2**: die in einer zweiten räumlichen Seitenansicht dargestellte Einrichtung gemäss Fig.1;
- **Fig.3**: die in grösserem Massstab sowie in Seitenansicht dargestellte Positioniereinheit für eine an einer Trägereinheit angeordneten Sonde, welche in bezug auf die optische Achse des Auges ausgerichtet ist;
- **Fig.4**: die gemäss Pfeilrichtung IV in Fig.3 in Ansicht dargestellte und an dem optischen Vermessungsgerät angeordnete Positioniereinheit;
- **Fig.5**: ein Teilstück des optischen Vermessungsgerätes mit einer Variante der Trägereinheit für die in den Projektionskonus ragende Sonde;
- **Fig.6**: ein in grösserem Massstab sowie im Schnitt dargestelltes Teilstück des Projektionskonusses mit der Sonde zum Indentieren der Cornea;
- **Fig.7**: das Teilstück gemäss Fig.6 mit der in den Strahlengang des Projektionskonusses hineinragenden und für die Indentation beispielsweise auf der theoretischen optischen Achse desselben angeordneten Sonde;
- **Fig.8**: ein in schematischer Ansicht sowie in grösserem Massstab dargestelltes Auge mit zugeordneter und teilweise dargestellter Sonde;
- **Fig.9**: das in Ansicht dargestellte Auge gemäss Fig.8 mit aufprojizierten Placido-Ringen sowie die für die Indentation beispielsweise in einer ersten Indentations-Position dargestellte Sonde;
- **Fig.10**: das Auge gemäss Fig.9 mit der beispielsweise in einer zweiten Indentations-Position dargestellten Sonde;
- **Fig.11**: das Auge gemäss Fig.9 mit der beispielsweise in einer dritten Indentations-Position dargestellten Sonde;
- **Fig.12**: die als Blockschema dargestellte Einrichtung zur Durchführung der Analyse und eine damit in Wirkverbindung stehende Auswerteinheit für die einzelnen Funktionsabläufe der Einrichtung; und
- **Fig.13**: die mit der erfindungsgemässen Einrichtung durchführbaren und als Fliessschema dargestellten Funktionsabläufe für die Analyse der Cornea.

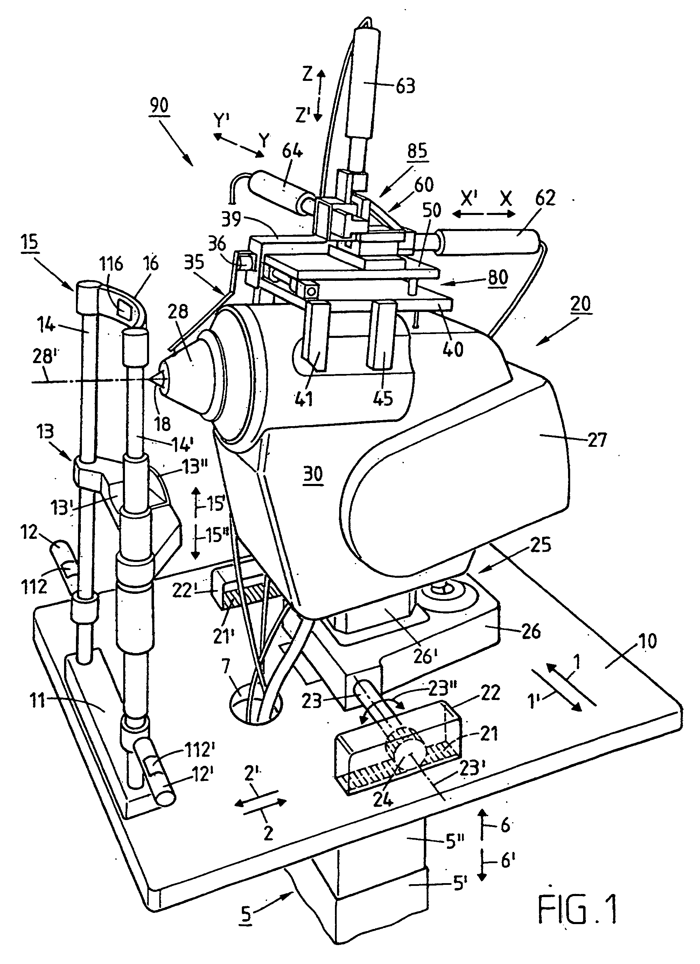

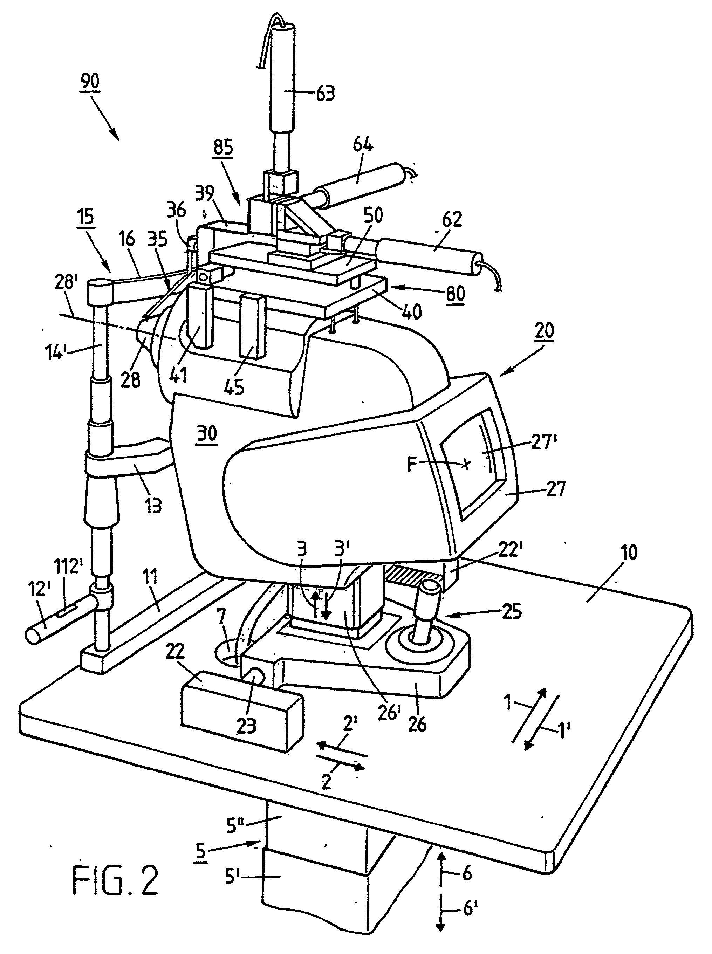

In Fig.1 und Fig.2 ist zur Verdeutlichung der Erfindung eine in der Gesamtheit mit 90 bezeichnete Einrichtung zur Durchführung von Analysen einer Cornea dargestellt, wobei die hier als Ausführungsbeispiel dargestellte Einrichtung 90 insbesondere zur Durchführung von Analysen am lebenden Auge (in vivo) ausgebildet ist. Es besteht jedoch auch die Möglichkeit mit der Einrichtung 90 eine Spenderhornhaut (in vitro) zu analysieren. Hierbei wird die jeweils zu analysierende Spenderhornhaut, vorzugsweise vor der Transplantation, mittels einer entsprechend ausgebildeten Halterung (nicht dargestellt) für eine Indentation der Einrichtung 90 zugeordnet.

An dieser Stelle wird darauf hingewiesen, dass die Einrichtung 90 zum Erfassen der momentanen (natürlichen) und der indentierten topologischen Beschaffenheit der Oberfläche der Cornea ein entsprechend ausgebildetes optisches Vermessungsgerät umfasst. Bei dem in Fig.1 und Fig.2 dargestellten Ausführungsbeispiel ist als optisches Vermessungsgerät zum Erfassen der topologischen Beschaffenheit der Cornea bespielsweise ein an sich bekanntes Keratoskop, vorzugsweise ein Videokeratoskop 20 vorgesehen.

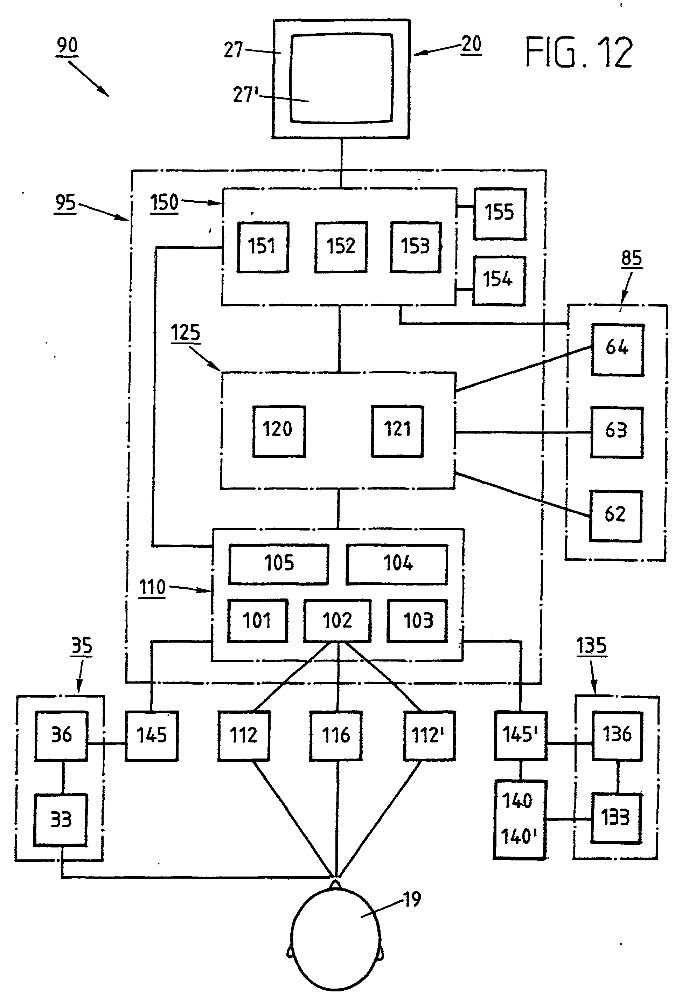

Die gemäss Fig.1 in einer ersten und gemäss Fig.2 in einer zweiten räumlichen Seitenansicht dargestellte Einrichtung 90 umfasst eine Standsäule 5, eine Tischplatte 10, eine Stützvorrichtung 15, das an sich bekannte Videokeratoskop 20 sowie eine daran angeordnete Positioniereinheit 85. Die mit elektromotorischen Antrieben 62,63 und 64 wirkverbundene Positioniereinheit 85 ist bei dem dargestellten Ausführungsbeispiel auf das Videokeratoskop 20 aufgesetzt und mit entsprechenden Schraubmitteln lösbar und auswechselbar daran befestigt. Die Positioniereinheit 85 sowie die damit wirkverbundenen Funktionselemente können somit auch an andere Keratoskope beziehungsweise analoge Geräte angeordnet werden. Die im wesentlichen mit den Funktionselementen 10,15,20 und 85 versehene Einrichtung 90 sowie eine damit in Wirkverbindung stehende Auswerteinheit 95 (Fig.12) werden nachstehend beschrieben.

Bei dem in Fig.1 und Fig.2 dargestellten Videokeratoskop 20 handelt es sich beispielsweise um eine bekannte Ausführungsform (KERATRON®, Optikon/Rom), wobei jedoch für die vorliegende Erfindung wie vorstehend erwähnt auch andere an sich bekannte optische Vermessungsgeräte Anwendung finden können. Derartige Keratoskope arbeiten beispielsweise nach der an sich bekannten Placido-Methode, wobei aber auch andere Verfahren und Systeme, wie beispielsweise die Rasterstereo-Photogrammetrie oder das Interferenzverfahren bei dem Analyseverfahren Anwendung finden können.

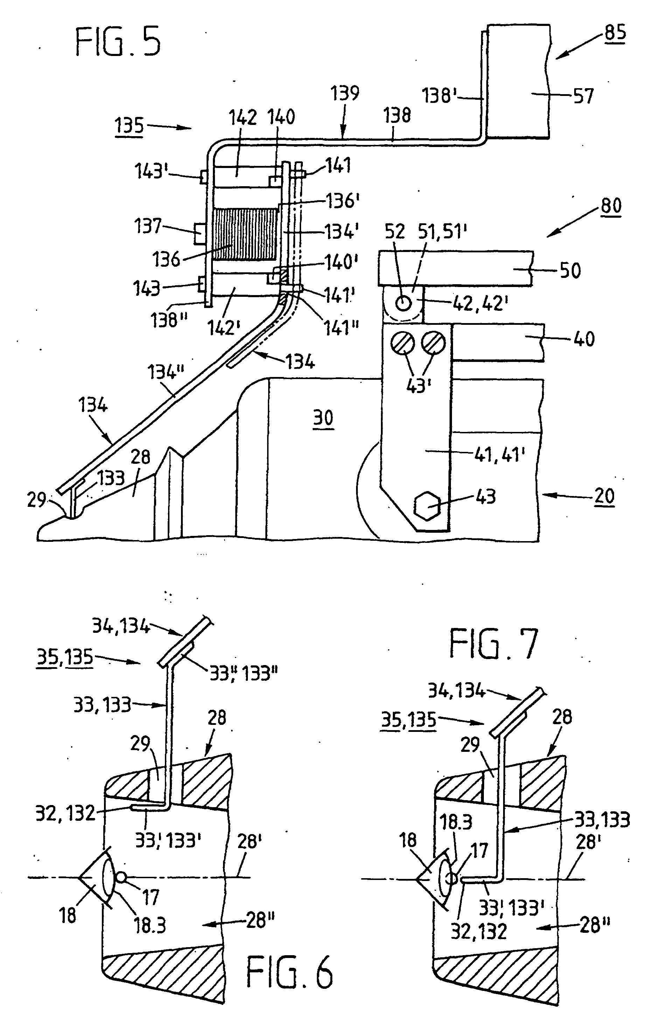

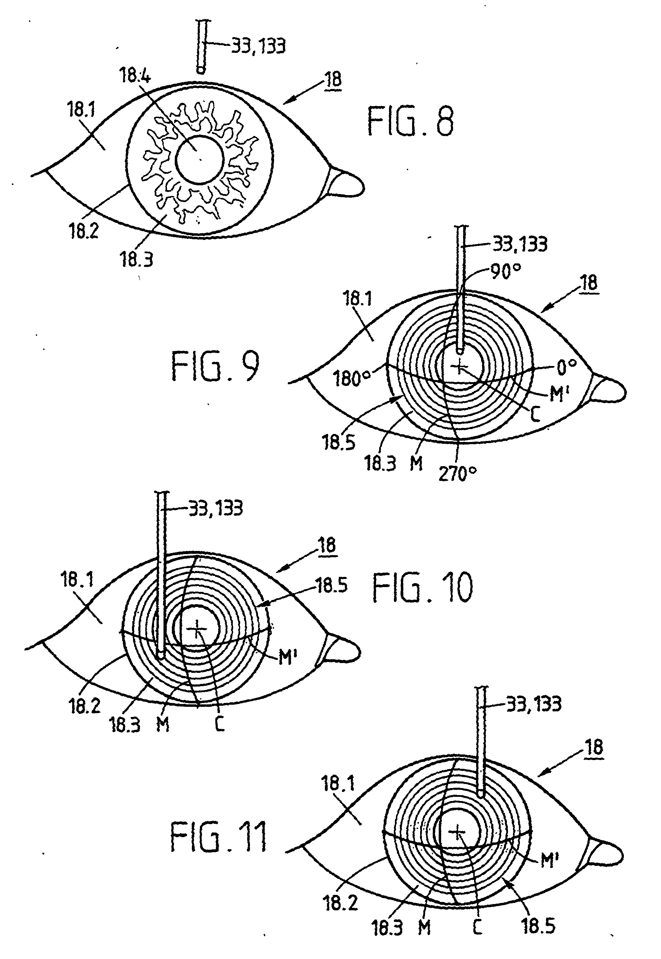

Bei diesem Anwendungsbeispiel arbeitet das Videokeratoskop 20 nach der an sich bekannten Placido-Methode, bei welcher mittels einer mit lichtundurchlässigen Ringen versehenen transparenten Scheibe mit zugeordneter Lichtquelle (nicht dargestellt) oder aber mittels eines am Videokeratoskop 20 angeordneten Projektionskonusses 28 mit zugeordneter Lichtquelle eine Anzahl Ringe auf die Vorderfläche der zu untersuchenden Cornea des Auges 18 projiziert beziehungsweise aufgeblendet werden. Bei der vorliegenden Erfindung ist das Videokeratoskop 20 mit dem als Messkopf ausgebildeten Projektionskonus 28 versehen. Die Ringe sind bei dem Projektionskonus 28 in bezug auf die theoretische optische Achse 28' (Fig.6,7) desselben konzentrisch und äquidistand dazu angeordnet. Die formgebende Ausgestaltung (visuelle Aufnahmen) der auf die Cornea 18.3 projizierten Ringe (Fig.9 bis Fig.11) ändert sich dabei in Abhängigkeit von der topologischen Beschaffenheit der jeweils zu analysierenden Cornea.

Die zur Aufnahme der Funktionselemente 15,20 und 85 ausgebildete Tischplatte 10 ist in nicht näher dargestellter Weise an der schematisch dargestellten Standsäule 5 angeordnet und befestigt. Die beispielsweise im Profilquerschnitt kastenförmig ausgebildete Standsäule 5 hat ein in nicht dargestellter Weise am Boden abgestütztes erstes Teilstück 5' sowie ein teleskopartig darin geführtes sowie mit nicht dargestellten Mitteln an der Tischplatte 10 befestigtes zweites Teilstück 5". Die Standsäule 5 kann entweder manuell oder mittels eines nicht dargestellten, hydraulisch oder pneumatisch betätigbaren Hubaggregats derart wirkverbunden sein, dass das zweite Teilstück 5" zusammen mit der daran befestigten Tischplatte 10 sowie den darauf angeordneten Funktionselementen 15,20 und 85 relativ zu dem ersten Teilstück 5' gemäss Pfeilrichtung 6 oder 6' höhenverstellbar sind. Die Tischplatte 10 ist weiterhin zur Durchführung nicht näher bezeichneter Versorgungskabel oder dergleichen mit einer Ausnehmung 7 versehen.

Die Stützvorrichtung 15 umfasst eine an der Tischplatte 10 mit nicht dargestellten Mitteln befestigte Halterung 11, zwei im Abstand zueinander daran angeordnete Führungsstangen 14 und 14', zwei Handgriffe 12 und 12', eine Kinnauflage 13 sowie eine am oberen Ende der Führüngsstangen 14,14' angeordne-te Stirnstütze 16. Die Stirnstütze 16 ist vorzugsweise in Richtung des am Videokeratoskop 20 angeordneten Projektionskonusses 28 konvex, bogenförmig ausgebildet. Die mit einem entsprechend geformten Auflageteil 13' und einem Anlagesteg 13" versehene Kinnauflage 13 ist an den beiden Führungsstangen 14, 14' relativ zu der Tischplatte 10 in Pfeilrichtung 15' beziehungsweise 15" manuell oder elektromotorisch verstellbar sowie in der jeweils erforderlichen Position feststellbar. Mit der in Pfeilrichtung 15' oder 15" vertikal orientierten Verstellbewegung wird erreicht, dass die Position der Kinnauflage 13 jeweils in Abhängigkeit des zu untersuchenden Patienten einstellbar ist. Hierdurch wird zudem gewährleistet, dass die theoretische optische Achse 28' des Projektionskonusses 28 für die Analyse der Cornea exakt in bezug auf den Patienten beziehungsweise auf das Auge 18 desselben einstellbar ist. Die Angleichung der theoretischen optischen Achse des jeweiligen Auges in bezug auf die optische Achse 28' des Projektionskonusses 28 erfolgt über einen im Zentrum desselben vorgesehenen Fixationspunkt (nicht dargestellt), auf welchen der Patient bei der Untersuchung blickt.

Wie in Fig.1 dargestellt ist an den beiden Handgriffen 12 und 12' jeweils ein Sensor 112 und 112' und an der Stirnstütze 16 ein weiterer Sensor 116 angeordnet. Die Funktions- und Wirkungsweise der schematisch dargestellten Sensoren 112 und 112' sowie 116 wird später in Verbindung mit der Auswerteinheit 95 (Fig.12) beschrieben.

Das Videokeratoskop 20 umfasst im dargestellten Ausführungsbeispiel ein mit dem Projektionskonus 28 sowie mit einem Monitor 27 und Bildschirm 27' (Fig.2) versehenes Gehäuse 30. An dem Gehäuse 30 ist ein mit einer vorzugsweise teleskopförmig verstellbaren Stütze 26' versehenes Tragteil 26 angeordnet, mittels welchem das Videokeratoskop 20 auf der Tischplatte 10 abgestützt ist. Weiterhin erkennt man in Fig.1 eine im Tragteil 26 gelagerte Welle 23, welche an den beiden Enden mittels daran angeordneter Laufrollen 24,24' (in Fig.1 ist nur die eine Laufrolle 24 dargestellt) oder dergleichen in entsprechend an der Tischplatte 10 befestigten Schienen 22 und 22' geführt ist. Die Welle 23 kann mit einem in dem Tragteil 26 angeordneten elektromotorischen Antrieb (nicht dargestellt) derart wirkverbunden sein, dass das Videokeratoskop 20 für eine fokussierende Bewegung auf der Tischplatte 10 relativ zu der Stützvorrichtung 15 beziehungsweise zu dem Patienten in Pfeilrichtung 2 oder 2' sowie an der Welle 23 gemäss Pfeilrichtung 1 oder 1' verschiebbar ist. Bei dem in Fig.1 schematisch dargestellten Ausführungsbeispiel sind die an der Welle 23 angeordneten Laufrollen 24,24' beispielsweise mit einer nicht bezeichneten Aussenverzahnung versehen und stehen jeweils mit einer in den Schienenkörpern 22,22' angeordneten Zahnstange 21,21' in Wirkverbindung. Die Welle 23 kann durch geeignete Mittel um ihre Längsachse 23' in Pfeilrichtung 23" drehbar angetrieben werden, so dass das Videokeratoskop 20 in Abhängigkeit der Drehbewegung entweder in Pfeilrichtung 2 oder 2' relativ zu der Stützvorrichtung 15 auf der Tischplatte 10 verschiebbar ist.

An dem Tragteil 26 ist ein Steuergriff 25 (JOYSTICK) angeordnet, mittels welchem die in Pfeilrichtung 1 oder 1' beziehungsweise 2 oder 2' orientierte Horizontalverstellung sowie die in Pfeilrichtung 3 oder 3' (Fig.2) orientierte Höhenverstellung des Videokeratoskops 20 durchführbar ist. Mit dem Steuergriff 25 (JOYSTICK) ist das Videokeratoskop 20 im wesentlichen hinsichtlich der vorstehend angegebenen Koordinaten verstellbar und dabei mit dem Projektionskonus 28 dem Auge 18 beziehungsweise der Cornea 18.3 (Fig.7) des jeweiligen Patienten zuführbar.

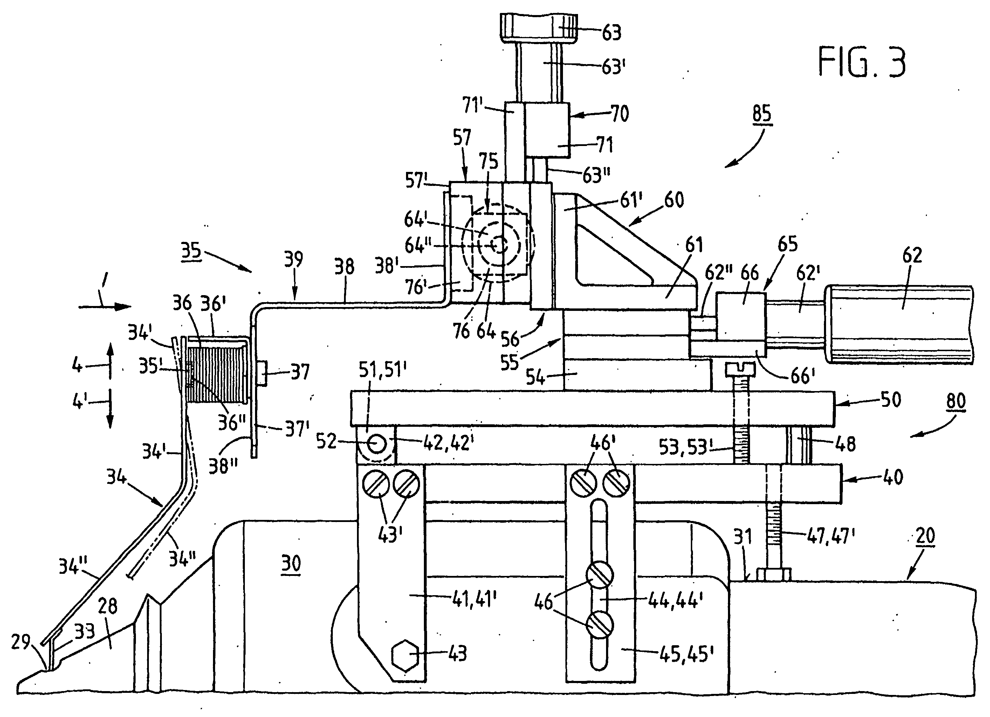

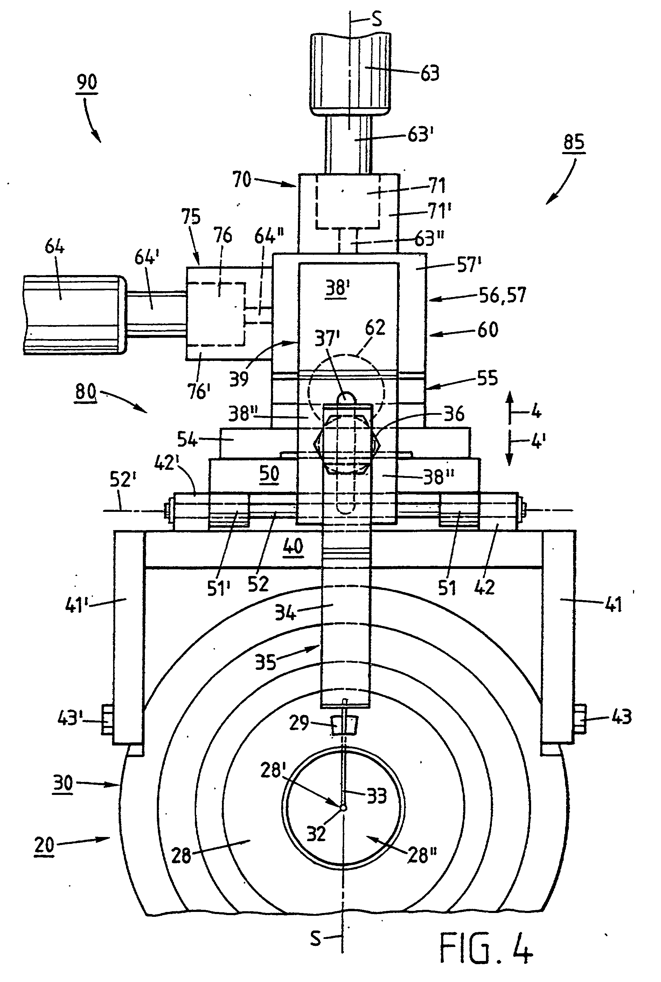

Die in Fig.1 und in Fig.2 schematisch dargestellte und beispielsweise auf dem Videokeratoskop 20 aufgesetzte Positioniereinheit 85 umfasst eine mit mehreren Funktionselementen versehene Trägereinheit 80 sowie die damit wirkverbundenen elektromotorischen Antriebe 62,63 und 64. Bei den in Fig.1 und Fig.2 schematisch dargestellten elektromotorischen Antrieben 62,63 und 64 handelt es sich beispielsweise um an sich bekannte Mikromotoren. Die Positioniereinheit 85 umfasst weiterhin eine mit den elektromotorischen Antrieben 62,63 und 64 wirkverbundene Verstellvorrichtung 60, an welcher an der dem Patienten beziehungsweise der Stützvorrich-tung 15 zugewandten Seite ein erster Tragbügel 39 und daran eine in der Gesamtheit mit 35 bezeichnete Indentationseinheit für eine Sonde 33 (Fig.3) angeordnet ist. Die in Fig.1 und Fig.2 sche-matisch dargestellte Positioniereinheit 85 mit den einzelnen damit wirkverbundenen Funktionselementen 60 und 35 wird nachstehend anhand der Figuren 3 und 4 im einzelnen beschrieben.

Fig.3 zeigt die in grösserem Massstab sowie in Seitenansicht dargestellte Positioniereinheit 85 sowie die Trägereinheit 80, welche im wesentlichen mittels einer ersten Platte 40 und daran angeordneter Stützen 41,41' und Stützen 45,45' an dem teilweise dargestellten Gehäuse 30 des Videokeratoskops 20 angeordnet ist. Die Stützen 41,41' und 45,45' sind jeweils durch Schrauben 43,43' und 46,46' an dem Gehäuse 30 sowie an der ersten Platte 40 befestigt. Am hinteren Ende der ersten Platte 40 sind vorzugsweise zwei im Abstand zueinander angeordnete und am Gehäuse 30 abgestützte Stellschrauben 47,47' vorgesehen, mittels welcher die Platte 40 relativ zu der Oberfläche 31 des Gehäuses 30 einstellbar und mittels der in Längsschlitzen 44,44' der hinteren Stützen 45,45' angeordneten Schrauben 46 feststellbar ist. Am vorderen Ende der ersten Platte 40 sind weiterhin zwei im Abstand zueinander angeordnete Lagerteile 42,42' vorgesehen, in welchen ein Bolzen 52 gelagert ist.

Im Abstand zu der ersten Platte 40 ist eine zweite Platte 50 angeordnet (Fig.3), welche am vorderen Ende mittels daran angeordneter Laschen 51,51' an dem Bolzen 52 und den Lagerteilen 42,42' der ersten Platte 40 angelenkt ist. Im hinteren Bereich sind weiterhin zwei quer zur Längsrichtung der zweiten Platte 50 beabstandete Stellschrauben 53,53' vorgesehen, mittels welcher die zweite Platte 50 in bestimmtem Bereich um die Längsachse 52' (Fig.4) des Bolzens 52 relativ zu der ersten Platte 40 durch eine Schwenkbewegung einstellbar ist. Die zweite Platte 50 ist vorzugsweise parallel zu der ersten Platte 40 einstellbar und mittels der Stellschrauben 53,53' feststellbar. Zwischen den beiden Platten 40 und 50 ist im hinteren Bereich vorzugsweise ein federelastisch wirkendes Distanzstück 48 angeordnet.

An der zweiten Platte 50 der Trägereinheit 80 ist zur Aufnah-me und Befestigung der Positioniereinheit 85 ein Zwischenstück 54 angeordnet, welches mittels einer nicht dargestellten Schraubverbindung an der zweiten Platte 50 befestigt ist. An dem Zwischenstück 54 ist ein mit einem Nabenteil 66 sowie einer Platte 66' versehener erster Lagerbock 65 angeordnet. Der erste Lagerbock 65 ist zur Aufnahme und Lagerung des ersten elektromotorischen Antriebs 62 ausgebildet, welcher mit einem Teilstück 62' in dem Nabenteil 66 gelagert und durch einen Mitnehmer 62" mit einem ersten Stellglied 55 wirkverbunden ist. Das erste Stellglied 55 ist mittels einer nicht dargestellten Schraubverbindung auswechselbar an dem Zwischenstück 54 befestigt.

Weiterhin erkennt man in Fig.3 die Verstellvorrichtung 60, welche ein mit einem ersten und zweiten Schenkel 61', 61" versehenes Winkelstück 61 hot und mit dem ersten Schenkel 61' an dem ersten Stellglied 55 angeordnet und mit einer nicht dargestellten Schraubverbindung oder dergleichen befestigt ist. An dem zweiten Schenkel 61" ist ein mit einem Nabenteil 71 und einer Platte 71' versehener zweiter Lagerbock 70 angeordnet, welcher zur Aufnahme (Lagerung) des zweiten elektromotorischen Antriebs 63 ausgebildet ist. Der zweite Antrieb 63 ist mit einem Teilstück 63' in dem Nabenteil 71 gelagert und durch einen Mitnehmer 63'' mit einem entsprechend zugeordneten und am Winkelstück 61 befestigten zweiten Stellglied 56 wirkverbunden. Dem zweiten Stellglied 56 ist ein mit dem dritten elektromotorischen Antrieb 64 in Wirkverbindung stehendes drittes Stellglied 57 zugeordnet. An dem dritten Stellglied 57 ist ein mit einem Nabenteil 76 und einer Platte 76' versehener dritter Lagerbock 75 angeordnet. Der dritte Antrieb 64 ist weiterhin mit einem Teilstück 64' in dem Nabenteil 76 gelagert und durch einen Mitnehmer 64'' mit dem dritten Stellglied 57 wirkverbunden (Fig.4).

Die vorstehend erwähnten und mit den schematisch dargestellten Stellgliedern 55,56 und 57 in Wirkverbindung stehenden elektromotorischen Antriebe 62,63 und 64 sind beispielsweise als eine Baueinheit auf der zweiten Platte 50 angeordnet. Mittels der Antriebe 62,63,64 werden die hochpräzisen und im µm-Bereich liegenden sowie in Richtung der Koordinaten gemäss der Pfeilrichtungen X,X' und Y,Y' sowie Z,Z' (Fig.1) orientierten Bewegungen der einzelnen Stellglieder 55,56,57 sowie die analogen Bewegungen der damit wirkverbundenen Elemente 33,35,36 und 39 erreicht (Fig.1). Die auf der zweiten Platte 50 angeordnete Positioniereinheit 85 kann beispielsweise mittels der Stellschrauben 53,53' in bestimmtem Bereich um die Achse 52' (Fig.4) des Gelenkbolzens 52 relativ zu der ersten Platte 40 bewegt (geschwenkt) werden. Bei der um den Gelenkbolzen 52 orientierten Schwenkbewegung kann die an der Indentationseinheit 35 angeordnete und mit dem freien Ende zum Indentieren der Cornea 18.3 durch eine Ausnehmung 29 des Projektionskonusses 28 (Messkopf) ragende Sonde 33 in bezug auf die optische Achse 28' des Projektionskonusses 28 eingestellt werden (Fig.6,7).

An der Stirnseite 57' des dritten Stellgliedes 57 ist, wie in Fig.3 dargestellt, der beispielsweise im Profilquerschnitt etwa 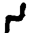 -förmig abgewinkelt ausgebildete erste Tragbügel 39 angeord-net. Der erste Tragbügel 39 hat ein erstes, horizontales Teilstück 38 und an dem einen Ende ein relativ dazu nach oben abgewinkeltes zweites Teilstück 38' und an dem anderen Ende ein nach unten abgewinkeltes drittes Teilstück 38". Der erste Tragbügel 39 ist mit dem zweiten Teilstück 38' an der Stirnseite 57' des dritten Stellgliedes 57 angeordnet und beispielsweise mittels einer nicht dargestellten Schraubverbindung lösbar befestigt. An dem dritten Teilstück 38" des ersten Tragbügels 39 ist ein Elektromagnet 36 sowie ein zweiter Tragbügel 34 angeordnet und in einem Längsschlitz 37' des ersten Tragbügels 39 in Pfeilrichtung 4 oder 4' verstellbar geführt sowie mittels einer Schraube 37 feststellbar.

Die in Fig.1 bis 4 als erste Variante dargestellte Indentationeinheit 35 umfasst den an einem Winkelstück 36' angeordneten und als Halteelement ausgebildeten Elektromagnet 36 sowie den zweiten Tragbügel 34 für die an dem einen Teilstück 34" angeordnete und befestigte Sonde 33, welche in Fig.3 teilweise dargestellt ist. An dem anderen abgewinkelten Teilstück 34' des zweiten Tragbügels 34 ist ein plättchenförmiges Zwischenstück 35' aus ferromagnetischem Material angeordnet, welches in zusammengebautem Zustand (Fig.3) in einer nicht näher dargestellten Ausnehmung 36" des Elektromagneten 36 angeordnet ist. Der zweite Tragbügel 34 sowie die daran angeordnete Sonde 33 sind vorzugsweise aus elektrisch leitendem Material hergestellt und in Abhängigkeit einer für die Indentation der Cornea einstellbaren Kraft an dem Elektromagnet 36 gehalten.

Der erforderliche Betriebsstrom für den Elektromagneten 36 ist vorzugsweise so einstellbar, dass der zweite Tragbügel 34 zusammen mit der Sonde 33 in Abhängigkeit einer vorbestimmten und entsprechend eingestellten Indentationskraft beim Überschreiten derselben automatisch durch eine Kippbewegung, wie in Fig.3 durch die Phantom-Linie (PHANTOMLINE) schematisch dargestellt, aus den Wirkungsbereich des Elektromagneten 36 bewegt und dadurch mindestens die Sondenspitze 32 (Fig.6,7) in nicht dargestellter Weise ausser Eingriff der Cornea 18.3 gelangt. Hierdurch wird auch bei etwaigen Fehlfunktionen der Auswerteinheit 95 (Fig.12) beziehungsweise der kompletten Einrichtung 90 die erforderliche Sicherheit des Patienten gewährleistet.

Fig.4 zeigt die gemäss Pfeilrichtung IV in Fig.3 in Ansicht dargestellte Einrichtung 90 mit der Positioniereinheit 85 und man erkennt das teilweise dargestellte Videokeratoskop 20 mit dem Gehäuse 30 und dem Projektionskonus 28 sowie die durch die Ausnehmung 29 desselben in den Strahlengang 28" ragende Sonde 33 mit der Kontaktspitze 32. Weiterhin erkennt man die vorderen Stützen 41,41' und Schrauben 43,43' der am Gehäuse 30 gelagerten und befestigten ersten Platte 40 sowie die mittels der Teile 42,42' und 51,51' und dem Bolzen 52 auf der ersten Platte 40 gelagerte zweite Platte 50 mit dem darauf angeordneten und mit nicht dargestellten Mitteln befestigten Zwischenstück 54. An dem Zwischenstück 54 ist das mit dem ersten elektromotorischen Antrieb 62 wirkverbundene erste Stellglied 55 angeordnet. Ferner erkennt man in Fig.4 die beiden anderen, an der Verstellvorrichtung 60 angeordneten Stellglieder 56 und 57 sowie die damit wirkverbundenen und in dem jeweiligen Lagerbock 70 und 75 gelagerten elektromotorischen Antriebe 63 und 64.

Bei der vorstehend als Ausführungsbeispiel beschriebenen Positioniereinheit 85 ist der dritte elektromotorische Antrieb 64 in bezug auf die in Fig.4 eingezeichnete Symmetrieachse S-S auf der einen Seite des damit wirkverbundenen dritten Stellgliedes 57 angeordnet. Es besteht jedoch auch die Möglichkeit, dass der dritte elektromotorische Antrieb 64 auf der anderen, gegenüberliegenden Seite der Symmetrieachse S-S angeordnet wird (nicht dargestellt). Bei einem weiteren Ausführungsbeispiel kann in nicht näher dargestellter Weise zusätzlich zu dem dritten Antrieb 64 an der anderen, gegenüberliegenden Seite ein vierter elektromotorischer Antrieb angeordnet werden.

Wie in Fig.4 dargestellt, ist an dem dritten Stellglied 57 der erste Tragbügel 39 der Indentationseinheit 35 angeordnet und in nicht näher dargestellter Weise befestigt. Die Indentationseinheit 35 umfasst weiterhin den vorzugsweise aus einem im Querschnitt rechteckig und relativ steif ausgebildeten Profilkörper (Fig.4) hergestellten zweiten Tragbügel 34 mit der daran angeordneten Sonde 33. Die Sonde 33 ist beispielsweise aus einem relativ dünnen Draht hergestellt. Für eine erste manuelle Einstellung der Sonde 33 ist die Indentationseinheit 35 zusammen mit den beiden Tragbügeln 34,39 relativ in bezug auf die theoretische optische Achse 28' (Fig.1) des Projektionskonusses 28 in Pfeilrichtung 4 oder 4' verschiebbar und mittels der Schraube 37 (Fig.3) feststellbar.

In Fig.5 ist als zweites Ausführungsbeispiel eine Indentationseinheit 135 mit daran angeordneter Sonde 133 für die Einrichtung 90 gemäss Fig.1 dargestellt und man erkennt ein Teilstück der am Videokeratoskop 20 angeordneten Trägereinheit 80 sowie ein Teilstück des Stellgliedes 57, welches mit den hier nicht dargestellten elektromotorischen Antrieben 62,63,64 (Fig.1-3) der Positioniereinheit 85 wirkverbunden ist. Weiterhin erkennt man die mittels der vorderen Stützen 41,41' am Gehäuse 30 des Videokeratoskops 20 befestigte erste Platte 40 sowie die zweite Platte 50, welche am vorderen Ende mittels der Laschen 51,51' an den Lagerteilen 42,42' der ersten Platte 40 angelenkt ist.

Die Indentationseinheit 135 hat ebenfalls einen im Profilquerschnitt beispielsweise 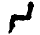 -förmig ausgebildeten ersten Tragbügel 139, welcher ein erstes, horizontales Teilstück 138, ein an dem einen Ende relativ dazu nach oben abgewinkeltes zweites Teilstück 138' sowie ein an dem anderen Ende nach unten abgewinkeltes drittes Teilstück 138" aufweist. Der erste Tragbügel 139 ist mit dem ersten Teilstück 138' an dem Stellglied 57 angeordnet und mit nicht dargestellten Mitteln befestigt. An dem dritten Teilstück 138" ist ein Elektromagnet 136 angeordnet, welcher bei diesem Ausführungsbeispiel durch eine Schraube 137 an dem dritten Teilstück 138" befestigt ist.

Abweichend von der Indentationseinheit 35 gemäss Fig.3 sind bei der Indentationseinheit 135 gemäss Fig.5 an dem dritten Teilstück 138" des ersten Tragbügels 139 zwei Haltebolzen 142,142' angeordnet. Die Haltebolzen 142,142' sind jeweils durch eine Schraubverbindung 143,143' oder dergleichen an dem dritten Teilstück 138" des ersten Tragbügels 139 befestigt. Am vorderen Ende des einzelnen Haltebolzens 142,142' ist ein Zapfen 141,141' angeformt sowie ein schematisch dargestellter Kraftsensor 140,140' angeordnet und mit nicht dargestellten Mitteln befe-stigt. Der einzelne Kraftsensor 140,140' ist vorzugsweise auswechselbar an dem Haltebolzen 142,142' angeordnet und befestigt.

Weiterhin erkennt man in Fig.5 einen abgekröpft ausgebildeten zweiten Tragbügel 134 für die Sonde 133. Der zweite Tragbügel 134 ist an dem ersten Teilstück 134' mit im Abstand zueinander angeordneten Bohrungen 141'' (nur einmal dargestellt) versehen und an den beiden Zapfen 141,141' der beiden Haltebolzen 142 und 142' gelagert. Der zweite Tragbügel 134 ist mit dem oberen Teilstück 134' in bezug auf den Elektromagnet 136 beispielsweise durch einen Luftspalt 136' im Abstand zum Elektromagnet 136 an den beiden Zapfen 141,141' der Haltebolzen 142,142' gelagert. An dem anderen Teilstück 134" des zweiten Tragbügels 134 ist die durch die Ausnehmung 29 in den Projektionskonus 28 des Videokeratoskops 20 ragende Sonde 133 angeordnet und in nicht näher dargestellter Weise befestigt. Der zweite Tragbügel 134 sowie die Sonde 133 sind beispielsweise aus elektrisch leitendem Material hergestellt und in Abhängigkeit einer für die Indentation der Cornea 18.3 (Fig.9-11) einstellbaren Kraft an dem Elektromagnet 136 gehalten.

Bei der Indentationseinheit 135 gemäss Fig.5 besteht die Möglichkeit, dass die eingestellte Kraft mittels der beiden Kraftsensoren 140,140' überwacht und beim Überschreiten derselben der zweite Tragbügel 134 aus dem Wirkungsbereich des Elektromagneten 136 gelangt und wie in Fig.5 durch die Phantom-Linie (PHANTOMLINE) schematisch dargestellt an den beiden Zapfen 141,141' geführt relativ zu dem Elektromagnet 136 beziehungsweise zu den beiden Haltebolzen 142,142' verschoben wird.. Hierdurch gelangt die Sonde 133 mit der Sondenspitze 132' ausser Eingriff der Cornea 18.3 des zu untersuchenden Auges 18 (Fig.7). Die Relativbewegung der Sondenspitze 132' in bezug auf die Cornea 18.3 liegt etwa in der Grössenordnung von 0,2 bis 0,5 mm.

Fig.6 und Fig.7 zeigt jeweils ein in grösserem Massstab sowie im Schnitt dargestelltes Teilstück des Projektionskonusses 28 sowie ein Teilstück der Indentationseinheit 35,135 mit dem Tragbügel 34,134. In Fig.6 ist die Indentationseinheit 35,135 mit der an dem Tragbügel 34,134 angeordneten und befestigten Sonde 33,133 für eine erste Abbildung der Topologie der Cornea 18.3 ausserhalb des Betrachtungsbereichs angeordnet. In Fig.7 ist die Indentationseinheit 35,135 mit der Sonde 33,133 in bezug auf die theoretische, optische Achse des schematisch dargestellten Auges 18 beispielsweise in beliebig gewählter Position dargestellt. Weiterhin erkennt man in Fig.6 und Fig.7 einen in dem Projektionskonus 28 (Messkopf) angeordneten und schematisch dargestellten Lichtsensor 17, vorzugsweise ein Infrarotsensor 17 sowie die zum Einführen der Sonde 33,133 im Projektionskonus 28 angeordnete Ausnehmung 29. Die Sonde 33,133 ist mit dem einen Ende 33",133" an dem Tragbügel 34,134 der Indentationseinheit 35,135 angeordnet und befestigt. Mit dem anderen Ende 33',133' ragt die Sonde 33,133 in den Strahlengang 28" des Projektionskonusses 28. Die Ausnehmung 29 in der Wand des Projektionskonusses 28 ist derart ausgebildet und bemessen, dass die Sonde 33,133 in bezug auf die theoretische Achse 28' des Projektionskonusses 28 horizontal in Längsrichtung desselben sowie in vorbestimmtem räumlichen Bereich verstellt werden kann. Die Sonde 33,133 kann somit der Cornea 18.3 für die Indentationen an einer Vielzahl beliebig wählbaren Stellen zugeführt werden.

Die dem Auge 18 zugewandte Spitze 32,132 der Sonde 33,133 ist zur Vermeidung einer Verletzung der Cornea 18.3 entweder mit einer nicht näher dargestellten absolut glatten Stirnfläche versehen, welche entweder flach oder abgerundet oder aber halbkugelförmig ausgebildet ist. Weitere Varianten bezüglich der Ausgestaltung der Spitze 32,132 und des Profilquerschnitts (Durchmesser oder dergleichen) der Sonde 33,133 sind ebenfalls möglich und liegen ebenfalls im Rahmen der vorliegenden Erfindung.

Mit den vorzugsweise aus oberflächlich elektrisch leitendem Material hergestellten Elementen 33,133 und 34,134 der Indentationseinheit 35,135 besteht die Möglichkeit, dass bei der jeweiligen Kontaktierung zeitlich sowie örtlich unterschiedliche Signale ermittelt und der Auswerteinheit 95 (Fig.12) zugeführt werden können. Die Sonde 33,133 ist vorzugsweise aus nichtrostendem Stahl hergestellt, so dass nach jedem Gebrauch eine desinfizierende Reinigung der Sonde 33,133 problemlos möglich ist. Von besonderem Vorteil ist, dass der zweite Tragbügel 34,134 mit der Sonde 33,133 ohne zusätzliche Hilfsmittel entfernt und somit die Reinigung der Sonde 33,133 ohne durchgeführt werden kann.

In Fig.8 ist in schematischer Ansicht sowie in grösserem Massstab das Auge 18 dargestellt und man erkennt die mit 18.1 bezeichnete Sklera, den mit 18.2 bezeichneten Limbus, die Cornea 18.3 sowie die mit 18.4 bezeichnete Pupille. Weiterhin ist in Fig.8 ein Teilstück der beispielsweise in der Ruhestellung (Fig.6) angeordneten Sonde 33,133 dargestellt.

Fig.9 zeigt das Auge 18 gemäss Fig.8 mit den in bezug auf das theoretische Zentrum C konzentrisch auf die Cornea 18.3 projizierten Placido-Ringen 18.5 sowie ein Teilstück der Sonde 33 oder 133, welche in dieser Stellung für die Indentation aus der Ruhestellung (Fig.8) in eine erste, beliebig gewählte Position bewegt wurde. Die Cornea 18.3 kann mittels der Sonde 33 oder 133 an einer oder aber an mehreren beliebig vorbestimmten Positionen nacheinander indentiert werden. Zur Erreichung der einzelnen Positionen wird die Sonde 33 oder 133 unter Einhaltung eines ausreichenden Abstandes zu der Cornea 18.3 entweder parallel zu dem ersten etwa senkrechten Meridian M zwischen 90° bis 270° oder aber etwa parallel zu dem zweiten horizontalen Meridian M' zwischen 0° bis 180° in ausreichendem Abstand zu der Cornea 18.3 bewegt. In den Figuren 10 und 11 ist das Auge 18 gemäss Fig.9 dargestellt, wobei abweichend davon die Sonde 33,133 gemäss Fig.10 in einer zweiten, beliebig gewählten und vorbestimmten Position und gemäss Fig.11 in einer dritten, beliebig gewählten und vorbestimmten Position dargestellt ist.

Ausgehend von dieser vorstehend anhand der Figuren 9 bis 11 beschriebenen ersten Phase wird in einer zweiten Phase die in den Strahlengang 28" des Projektionskonusses 28 ragende Sonde 33 oder 133 aus der im Abstand zur Cornea 18.3 angeordneten Position (Fig.7) linear in Richtung der theoretischen optischen Achse 28' bewegt und dem nicht näher bezeichneten Apex der Cornea 18.3 für die eigentliche Indentation zugeführt. Die von dem Augenchirurgen (Ophthalmologen) jeweils gewählte und örtlich vorbestimmte Indentation der Cornea 18.3 bewirkt im Bereich derselben eine örtliche, konkave Verformung. Die in Abhängigkeit von der Beschaffenheit der Cornea jeweils vorbestimmte Kraft N (Newton) und/oder die in mehreren Stufen, zum Beispiel in Stufen der Grössenordnung von 100, 200, 300, 400, 500 und 800 µ (MICRON) gewählte Vorschubdistanz für die Indentation wird vorzugsweise von dem Augenchirurgen (Ophthalmologen) derart vorgewählt und mit geeigneten Mitteln eingestellt, dass die Indentation programmgesteuert mittels der elektromotorischen Antriebe 62,63 und 64 ohne weitere Hilfsmittel automatisch erfolgt.

An dieser Stelle wird darauf hingewiesen, dass mittels der Positioniereinheit 85 die von dem ersten Antrieb 62 in Pfeilrichtung X', von dem zweiten Antrieb 63 in Pfeilrichtung Z' und von dem dritten Antrieb 64 in Pfeilrichtung Y orientierte Zustellbewegung sowie die jeweils in umgekehrter Pfeilrichtung X,Z und Y' orientierte Rückstellbewegung der Indentationseinheit 35,135 mit der Sonde 33, 133 erreicht wird.

Mit dem zweiten Antrieb 63 wird die während der ersten Phase etwa parallel zu dem etwa senkrechten Meridian M zwischen 90° bis 270° orientierte Bewegung und mit dem dritten Antrieb 64 die etwa parallel zu dem horizontalen Meridian M' zwischen 0° bis 180° orientierte Bewegung der Sonde 33,133 erreicht (Fig.9).

Mit dem ersten Antrieb 62 wird ausschliesslich die für die eigentliche Indentation der Cornea 18.3 und im wesentlichen in Richtung der theoretischen Achse 28' des Projektionskonusses 28 orientierte Zuführbewegung der Sonde 33,133 erreicht.

Die elektromotorischen Antriebe 62,63 und 64 werden für die einzelnen Bewegungsphasen vorzugsweise getrennt gesteuert und aktiviert. Nach Abschluss der Indentation wird die Sonde 33,133 mittels des ersten elektromotorischen Antriebs 62 gemäss Pfeilrichtung X (Fig.1) wieder von der Cornea 18.3 entfernt. Für eine weitere Indentation wird die Sonde 33,133 räumlich neu ausgerichtet und anschliessend wieder gemäss Pfeilrichtung X' (Fig.1) der Cornea 18.3 zugeführt. Die Steuerung der elektromotorischen Antriebe 62,63 und 64 ist dabei so geregelt, dass eine transversale Bewegung bei aufgesetzter und die Cornea 18.3 kontaktierender Sonde 33,133 ausgeschlossen ist.

In Fig.12 ist die komplette Einrichtung 90 zum Erfassen der Topologie der Cornea als Blockschaltbild dargestellt und man erkennt das schematisch dargestellte Videokeratoskop 20 (Topologie-Erfassungsgerät) mit dem Monitor 27 und Bildschirm 27' sowie die ebenfalls schematisch dargestellte und mit den drei elektromotorischen Antrieben 62,63 und 64 versehene Positioniereinheit 85. Weiterhin ist in Fig.12 die für die einzelnen Funktionsabläufe mit der Einrichtung 90 in Wirkverbindung stehende und in der Gesamtheit mit 95 bezeichnete Auswerteinheit dargestellt.

Im dargestellten Ausführungsbeispiel umfasst die Auswerteeinheit 95 eine Detektionseinheit 110 (DETEKTIONS UNIT) eine mit den elektromotorischen Antrieben 62,63 und 64 der Positioniereinheit 85 in Verbindung stehende und als sogenannte Schnittstelle vorgesehene Steuereinheit 125 (INTERFACE UNIT) sowie einen Prozessor 150 (COMPUTER). Die Steuereinheit 125 und der Prozessor 150 sind ebenfalls mit der Positioniereinheit 85 verbunden. Weiterhin steht der Prozessor 150 mit einem Drucker, vorzugsweise mit einem Farbdrucker 154 und einem Farbmonitor 155 in Verbindung. Die einzelnen Funktionseinheiten 110,125 und 150 werden nachstehend beschrieben. In dem Blockschaltbild gemäss Fig.12 sind ferner die beiden mit der Detektionseinheit 110 in Verbindung stehenden Handgriffsensoren 112 und 112' sowie der Kopfstützensensor 116 für den Patienten 19 dargestellt.

Bei einer ersten Variante gemäss Fig.12 steht die Detektionseinheit 110 unter Zwischenschaltung entsprechender Steuermittel 145 mit der vorstehend in Verbindung mit Fig.3 beschriebenen und an der Indentationseinheit 35 angeordneten Sonde 33 und dem Elektromagnet 36 in Wirkverbindung. Bei einer zweiten, ebenfalls in Fig.12 dargestellten Variante besteht die Möglichkeit, dass die Detektionseinheit 110 unter Zwischenschaltung entsprechender Steuermittel 145' mit der vorstehend in Verbindung mit Fig.5 beschriebenen und an der Indentationseinheit 135 angeordneten Sonde 133 und dem Elektromagnet 136 in Wirkverbindung steht. Bei dieser Variante ist dem Elektromagneten 136 zusätzlich mindestens einer der beiden Kraftsensoren 140 oder 140', vorzugsweise aber beide Kraftsensoren 140,140' zugeordnet. Mittels der erwähnten Steuermittel 145 oder 145' kann der an dem zugeordneten Elektromagnet 36 oder 136 anliegende Strom durch entsprechende Signale entweder fix eingestellt oder aber von dem Prozessor 150 infolge einer Vorwahl reguliert und eingestellt werden.

An dieser Stelle wird darauf hingewiesen, dass die Detektionseinheit 110 gemäss der ersten Variante entweder mit den Elementen 33,35,36 und 145 (Fig.3) oder aber gemäss der zweiten Variante mit den Elementen 133,135,136 und 140,140' sowie 145' (Fig.5) sowie mit dem Prozessor 150 elektrisch wirkverbunden ist.

Die in Fig.12 schematisch dargestellte Detektionseinheit 110 ist ein wesentlicher Bestandteil zur Durchführung einzelner Funktionsabläufe. Die mit den Handgriffsensoren 112,112' und dem Kopfstützensensor 116 in Verbindung stehende Detektionseinheit 110 umfasst mehrere elektronische Komponenten 101 bis 105, welche durch nicht dargestellte elektrische Mittel entsprechend miteinander verbunden sind. Im dargestellten Ausführungsbeispiel umfasst die Detektionseinheit 110 mindestens einen Oszillator 101, mindestens einen Verstärker 102, mindestens einen Gleichrichter 103 und mindestens einen Frequenzfilter 104 sowie mindestens einen Vergleicher 105 (Comparator).

Für eine sogenannte Patientenerkennung wird ein Signal, beispielsweise ein tonfrequentes Signal mit einer Spannung von 12 Volt und einer Frequenz von etwa 4500 Hertz erzeugt und an einem der Handgriffe 12 oder 12' angelegt oder mit geeigneten Mitteln, beispielsweise mit einem nicht dargestellten Ohrclip oder EKG-Kontakt am Patienten angeschlossen. Der Anschluss mittels des Ohrclips beziehungsweise EKG-Kontakts wird insbesondere bei Patienten mit fehlenden Gliedmassen verwendet.

Die Detektionseinheit 110 ist weiterhin mit nicht näher dargestellten elektronischen Elementen versehen, mittels welcher der jeweilige Kontakt der Sondenspitze 32 oder 132 an der Cornea 18.3 (Fig.7) erfasst wird. Weiterhin werden von dem Prozessor 150 in Verbindung mit der Detektionseinheit 110 die bei der Indentation bewirkten Cornea-Topologien in Abhängigkeit der verschiedenen Indentationsstufen, beispielsweise beim Erstkontakt von 100;200;300;400;500 oder 800 µ, pulssynchron erfasst und gespeichert.

Die sequentiell erfassten Daten der Cornea-Topologien werden vorzugsweise in der gleichen Phase des Herzschlages des jeweiligen Patienten erfasst und dadurch etwaige herzschlagabhängige Verfälschungen verhindert. Bei Berührung der beiden Sensoren 112 und 112' oder der Sensoren 112' und 116 oder der Sensoren 112' und der Sonde 33,133 durch den Patienten werden entsprechende Signale von der Detektionseinheit 110 erfasst, gefiltert und mit zwei oder mehreren verschiedenen Pegeln verglichen. Aus diesen Vergleichen können die folgenden Zustände "Aktiv" oder "Inaktiv" beziehungsweise "Fehlerhaft" ermittelt und als entsprechend verarbeitete Signale dem Prozessor 150 zugeführt werden. Diese selbstdiagnostischen Zustände gewährleisten weitere, erforderliche Sicherheitsansprüche an die erfindungsgemässe Einrichtung 90. Bei etwaigem Defekt wird das Steuerungsprogramm derart aktiviert, dass eine bereits laufende Untersuchung unterbrochen wird beziehungsweise umgehend manuell unterbrochen werden kann. Die Sicherheitskomponenten des beschriebenen Systems deaktivieren bei etwaigem Systemfehler oder unerwünscht hohem beziehungsweise niedrigem Messwert von Kraft, Puls etc, den Haltemagneten 36 (Fig.3) oder 136 (Fig.5), wodurch die laufende Untersuchung sofort unterbrochen wird.

Zur Erreichung der Selbstdiagnose ist die Detektionseinheit 110 mit zusätzlichen elektronischen Mitteln (nicht dargestellt) versehen, welche einerseits den Kontakt der Sonde 33 oder 133 an der Cornea 18.3 erfasst und andererseits etwaige Fehlfunktionen erkennt. Derartige Fehlfunktionen, in Form extensiver Kraft auf die Cornea 18.3 des Auges 18 oder fehlendem Kontakt mit der Cornea 18.3 beziehungsweise kippender Sonde 33 oder 133 oder dergleichen, können beispielsweise durch entsprechende Signale zur Weiterverarbeitung dem Prozessor 150 zugeführt und/oder für den Augenchirurgen (Ophthalmologen) an dem Bildschirm 27 als entsprechende Information angezeigt werden.

Die in Fig.12 schematisch dargestellte Steuereinheit 125 (INTERFACE UNIT) steht mit den drei elektromotorischen Antrieben 62,63 und 64 der Positioniereinheit 85 sowie mit dem Prozessor 150 in Wirkverbindung. Die Steuereinheit 125 umfasst im wesentlichen einen Regler 120 sowie einen sogenannten Treiber 121. Mittels der Komponenten 120 und 121 können die verschiedenen Signale für die Funktion der gesamten Auswerteinheit 95 aufbereitet und weitergeleitet werden.

Der Prozessor 150 umfasst im wesentlichen eine Bilderfassungseinheit 151 (FRAMEGRABBER), eine Regeleinheit 152 (Converter) sowie ein Steuermodul 153 (Controller). Die Bilderfassungseinheit 151 ist vorzugsweise mit nicht dargestellten optronischen (optisch/elektronisch) Sensoren versehen, mittels welcher die während der einzelnen Indentationen aufgenommenen Abbildungen der Topologien der Cornea 18.3 erfasst und beispielsweise für die Weiterverarbeitung gespeichert werden. Die Regeleinheit 152 dient zur Auswertung der von den Sensoren zugeführten Signale sowie zur Einstellung des erforderlichen elektrischen Stroms für den als Halterung für die Sonde 33 oder 133 ausgebildeten Elektromagnet 36 oder 136. Das Steuermodul 153 dient zur Funktionskontrolle der elektromotorischen Antriebe 62,63 und 64 sowie zur Datenerfassung der Detektionseinheit 110. Der Prozessor 150 steht weiterhin mit einem Drucker, vorzugsweise mit einem Farbdrucker 154 in Verbindung. Von dem Farbdrucker 154 werden die von einer nicht dargestellten Kamera erfassten Topologien der Cornea 18.3 vorzugsweise in Form einzelner oder zusammenhängend bewegter Abbildungen (Einzelbilder, Film, Nativ-Fotos, Falschfarbfotos oder dergleichen) ausgedruckt. Der Prozessor 150 steht zudem mit einem Farbmonitor 155 in Verbindung, von welchem die in dem Prozessor 150 ablaufenden Funktionen vorzugsweise farblich, visuell dargestellt werden können.

Die Arbeitsweise der erfindungsgemässen Einrichtung 90 zum Erfassen der Topologie der Cornea für die Analyse wird nachstehend beschrieben:

In einer ersten Phase wird von dem Untersuchenden bei aktivierter Einrichtung 90 das damit in Wirkverbindung stehende Videokeratoskop 20 mit dem Projektionskonus 28 in bezug auf das zu untersuchende Auge 18 (Fig.1) des jeweiligen Patienten hinsichtlich der Lage und Entfernung derart positioniert, dass ein in Fig.2 am Bildschirm 27' des Videokeratoskops 20 angezeigtes Fadenkreuz F (einblendbar) mit dem in den Figuren 9,10 und 11 mit C bezeichneten theoretischen Zentrum des Auges 18 deckungsgleich ist.

In Abhängigkeit von der zu analysierenden Cornea 18.3 wird von dem Augenchirurgen (Ophthalmologen) die örtliche Position sowie Tiefe der durchzuführenden Indentation und/oder Indentationskraft beispielsweise in Schritten bestimmt und anschliessend mittels der programmgesteuerten elektromotorischen Antriebe 62,63 und 64 die Indentationseinheit 35 oder 135 mit der daran befestigten Sonde 33 oder 133 in eine vorgewählte Ausgangsposition, wie beispielsweise in Fig.9 oder Fig.10 beziehungsweise Fig.11 schematisch dargestellt, transportiert.

Voraussetzung für die eingangs erwähnte dynamische Analyse der Cornea 18.3 (Dynamic Corneal Imaging = DCI) besteht in der exakten Positionierung des jeweiligen Patienten, welcher mit mindestens einer Hand den Handgriffsensor 112 oder 112' sowie mit der Stirn den an der Stirnstütze 16 angeordneten Kontaktsensor 116 (Fig.1) aktiviert. Weiterhin wird von dem Augenchirurgen (Ophthalmologen) das Videokeratoskop 20 mit dem Projektionskonus 28 in eine sogenannte Fokusposition gebracht. Dabei durchdringt die Cornea 18.3 des jeweiligen Patienten, wie in Fig.7 schematisch dargestellt, den im Projektionskonus 28 von dem Lichtsensor 17 erzeugten Lichtstrahl (nicht dargestellt) etwa bis zur Hälfte desselben. Hierdurch wird die für die Auswertung erforderliche Grösse und Schärfe der vor und bei der Indentation jeweils von der Cornea 18.3 erfassten Abbildungen (Fotos) gewährleistet.

Eine erste Abbildung (Aufnahme, Foto etc.) von der zu indentierenden und zu analysierenden Cornea 18.3 kann wahlweise ohne die in den Projektionskonus 28 ragende Sonde 33,133 beziehungsweise zusammen mit der Sonde 33,133 aufgenommen werden. Bei der ersten Variante wird die in der Ausgangsposition noch im Abstand zur Cornea 18.3 angeordnete Sonde 33,133, wie beispielsweise in Fig.6 und Fig.8 dargestellt, in bezug auf die optische Achse 28' des Projektionskonusses 28 nach oben aus dem Sichtbereich der nicht dargestellten Kamera des Videokeratoskops 20 gefahren und anschliessend eine erste Abbildung (Foto etc.) der natürlichen Topologie der Cornea 18.3 erfasst und gespeichert. Bei der zweiten Variante wird die Sonde 33,133 bei bereits aktivierten Sensoren 112,112' beziehungsweise 116 von der Ausgangsposition in Richtung der optischen Achse 28' gefahren bis diese die Cornea 18.3 beziehungsweise den Tränenfilm derselben berührt. Dabei wird ein entsprechendes Signal ausgelöst und anschliessend die erste Abbildung der natürlichen Topologie der Cornea 18.3 erfasst und gespeichert.

Sobald die mit den geeigneten Mitteln erfasste und in Form von Signalen bestätigte Position der Sonde 33,133 erreicht ist, wird diese ausschliesslich von dem ersten Antrieb 62 (Fig.1) für die örtlich vorbestimmte Indentation in Richtung der Cornea 18.3 bewegt und anschliessend zur Erreichung der ebenfalls vorbestimmten Indentationstiefe und/oder Indentationskraft weiterbewegt. Nachdem die örtlich von der Sonde 33,133 bewirkte und mit geeigneten Mitteln überwachte Indentationstiefe und/oder Indentationskraft an der Cornea 18.3 erreicht ist, wird die Videokamera aktiviert und von der örtlich konkav eingedellten, momentanen Formgebung (Topologie) eine weitere Abbildung (Foto oder dergleichen) erfasst. Weitere Indentationspunkte an der Cornea 18.3 werden von der entsprechend vorbestimmt verstellbaren Sonde 33,133 nacheinander angefahren und entsprechend indentiert und dabei die jeweilige konkave Indentation erfasst, vorzugsweise fotographisch erfasst. Die von der Sonde 33,133 bewirkte Indentationstiefe und/oder Indentationskraft kann von der motorabhängigen und gesteuerten Zustellbewegung der Sonde 33,133 beziehungsweise oder aber auch von der vorzugsweise mit geeigneten Mitteln einstellbaren und überwachbaren Kraft erfolgen. Die eingestellte Kraft wird von dem mit der Detektionseinheit 110 in Wirkverbindung stehenden Sonde 33,133 beziehungsweise Kraftsensor 140,140' (Fig.5 und Fig.12) überwacht.

Sobald sämtliche, beispielweise von dem Augenchirurgen vorbestimmt und programmabhängig an der Cornea 18.3 vorgegebenen Punkte von der Sonde 33,133 indentiert und daraus entsprechende Daten erfasst sind, wird die Sonde 33,133 wieder in die Ausgangsposition zurückgeführt. Beim Verlassen der Sonde 33,133 von der Cornea 18.3 kann eine visuelle Information am Farbmonitor 155 angezeigt werden und/oder ein oder mehrere akustische Signale erzeugt werden. Wesentlich ist es, dass für die Indentation der Cornea 18.3 ausschliesslich der erste Antrieb 62 gemäss der in Richtung der optischen Achse 28' orientierten Pfeilrichtung X' (Fig.1) aktiviert wird. Die beiden anderen Antriebe 63 und 64 sind in dieser Phase passiv, so dass eine transversale Bewegung der an der Cornea 18.3 anliegenden Sonde 33,133 ausgeschlossen ist.

Grundsätzlich wird im wesentlichen mittels der Einrichtung 90 die bei der Indentation von der Sonde 33,133 bewirkte Verformung und somit die momentane Topologie der Cornea 18.3 erfasst. Aus den einzelnen Topologien werden rechnergestützt und mathematisch verarbeitete Daten erzeugt, wobei die Daten in Form visueller oder aber akustischer Mittel gespeichert werden. Die visuellen und/oder akustischen Mittel können für die eigentliche Analyse der Cornea 18.3 entsprechend wiedergegeben (Reproduktion) werden.

Die aus den einzelnen Topologien der indentierten Cornea 18.3 rechnergestützt und mathematisch verarbeiteten Daten in Form akustischer Signale können im wesentlichen von dem Prozessor 150 in eine Reihe von Zahlen umgewandelt werden. Hierbei wird beispielsweise die Amplitude des Signals in regelmässigen oder unregelmässigen Zeitabständen gemessen und als Zahl gespeichert. Weiterhin besteht die Möglichkeit, die verschiedenen Anteile gegebener Wellen in dem zu analysierenden Signal in tiefe und hohe Töne zu zerlegen. Vorzugsweise wird aus den vorliegenden Informationen (Höhe und Krümmung etc.) entlang eines bestimmten Radius der Cornea 18.3 ein akustisches Signal erzeugt, wobei der entstehende Ton im Prozessor 150 (Computer) simuliert und wiedergegeben werden kann.

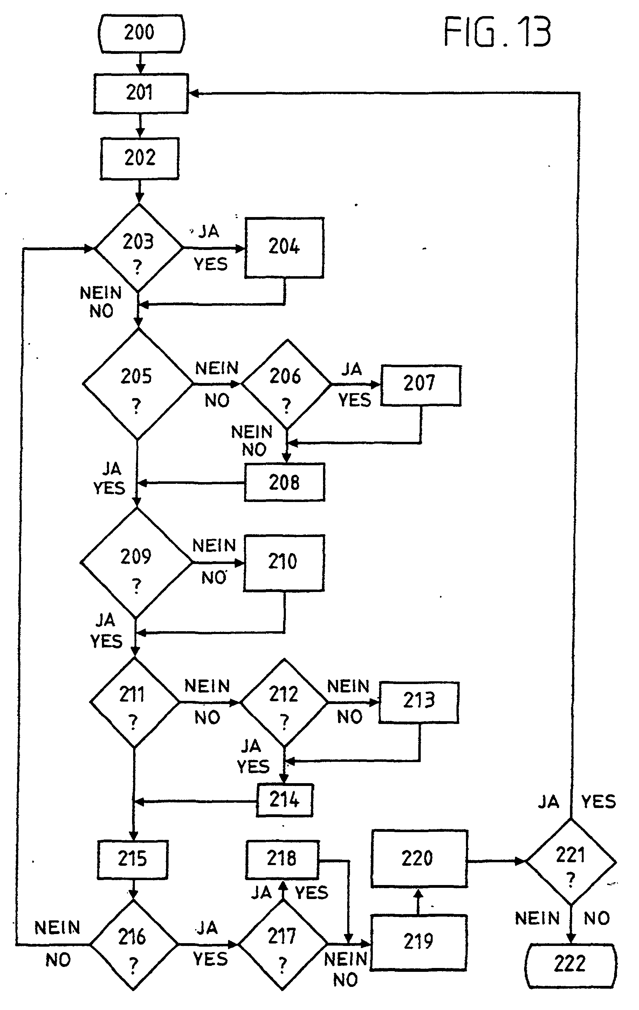

Die einzelnen mit der erfindungsgemässen Einrichtung durchführbaren Funktionsabläufe 200 bis 222 für die Analyse, insbesondere für die topologieabhängige Analyse der Cornea 18.3 erfolgen beispielsweise wie nachstehend in Verbindung mit Fig.13 im einzelnen beschrieben;
Schritt 200: Vorbereitung der mit dem optischen Vermessungsgerät beziehungsweise dem Videokeratoskop 20 elektronisch und programmgesteuert wirkverbundenen Einrichtung 90 für die Analyse der Cornea;
Schritt 201: Videokeratoskop 20 in den Betriebsmodus zum Erfassen topologischer Daten versetzen;
Schritt 202: Positionierung des Videokeratoskops 20 mit dem Projektionskonus 28 in bezug auf den jeweiligen Patienten beziehungsweise Positionierung des Patienten in bezug auf das Videokeratoskop 20;
Schritt 203: Beurteilung und Entscheid ob eine Abbildung (Foto etc.) von der natürlichen Topologie der zu untersuchenden Cornea 18.3 ohne die Sonde 33 oder 133 gemäss Fig.6 oder aber zusammen mit der Sonde 33 oder 133 gemäss Fig.7 aufzunehmen ist; wenn
   a) JA (YES): ohne die Sonde 33 oder 133, es folgt Schritt 204;
   b) NEIN (NO): mit der Sonde 33 oder 133, weiter zu Schritt 205;
Schritt 204: Aktivierung der beiden elektromotorischen Antriebe 63 und 64 für die in Pfeilrichtung Y und/oder Z (Fig.1) orientierte Bewegung der Sonde 33 oder 133 zur Erreichung der vorbestimmten Position gemäss Fig.6;
Schritt 205: Überprüfung der momentanen Position in bezug auf die Y und Z Koordinaten mit der für die nächste topologieabhängige Abbildung (Foto etc.) der Cornea 18.3 erforderliche Position der Sonde 33 oder 133; wenn
   a) JA (YES): weiter zu Schritt 209;
   b) NEIN (NO): es folgt Schritt 206
Schritt 206: Überprüfung ob die Sonde 33 oder 133 mit der Cornea 18.3 kontaktierend in Eingriff steht; wenn
   a) JA (YES): Kontakt vorhanden, weiter zu Schritt 207;
   b) NEIN (NO): Kontakt nicht vorhanden, es folgt Schritt 208;
Schritt 207: Aktivierung des Antriebs 62 zum Zurückziehen beziehungsweise Abheben der Sonde 33 oder 133 gemäss Pfeilrichtung X (Fig.1) relativ zu der Cornea 18.3;
Schritt 208: Aktivierung der beiden Antriebe 63,64 für die in Pfeilrichtung Y und Z (Fig.1) orientierte Verstellbewegung der Sonde 33 oder 133;
Schritt 209: Überprüfung ob die Sonde 33 oder 133 aufgesetzt und mit der Cornea 18.3 kontaktierend in Eingriff steht; wenn
   a) JA (YES): Kontakt vorhanden, weiter zu Schritt 211;
   b) NEIN (NO): Kontakt nicht vorhanden, es folgt Schritt 210;
Schritt 210: abwarten bis die Fokussierung des Videokeratoskops 20 abgeschlossen ist;
Schritt 211: Überprüfung und Entscheid ob die Abbildung (Foto etc.) der Topologie ohne die Sonde 33 oder 133 erfolgen soll; wenn
   a) Ja (YES): weiter zu Schritt 215;
   b) NEIN (NO): es folgt Schritt 212;
Schritt 212: Feststellen ob die Sonde 33 oder 133 aufgesetzt ist; wenn
   a) JA (YES): weiter zu Schritt 214;
   b) NEIN (NO): es folgt Schritt 213;
Schritt 213: Positionierung der Sonde 33 oder 133 und Kontaktierung beziehungsweise Aufsetzen der Sonde 33 oder 133 an die Vorderfläche der Cornea 18.3;
Schritt 214: Durchführung der Indentation der Cornea 18.3 an der vorbestimmten Stelle mit vorgegebenen Werten hinsichtlich der Kraft und/oder der Indentationstiefe;
Schritt 215: Erstellen der Abbildung (Foto etc.) der topologischen Beschaffenheit an den einzelnen, vorbestimmten Stellen hinsichtlich der durch die Indentation momentan bewirkten Verformung der Cornea 18.3;
Schritt 216: Überprüfung ob die vorbestimmten und für die Analyse der Topologie der Cornea 18.3 erforderlichen Indentationen erfolgt sind; wenn
   a) JA (YES): weiter zu Schritt 217;
   b) NEIN (NO): Rückführung zu Schritt 203 und nochmalige Durchführung der einzelnen, vorstehend genannten Funktionsabläufe 203 bis 215;
Schritt 217: Überprüfung ob die Sonde 33 oder 133 noch mit der Cornea 18.3 kontaktierend in Eingriff steht; wenn
   a) JA (YES): weiter zu Schritt 218;
   b) NEIN (NO): es folgt Schritt 219;
Schritt 218: die Sonde 33 oder 133 wird durch Aktivierung des Antriebs 62 gemäss Pfeilrichtung X (Fig.1) relativ zu der Cornea 18.3 zurückgezogen;
Schritt 219: Beurteilung und gegebenenfalls Speicherung der bei der einzelnen Indentation der Cornea 18.3 in Abhängigkeit der Topologie gewonnenen Daten in Form einzelner oder zusammenhängender Abbildungen (Fotos, Film etc.) beziehungsweise in Form akustischer Signale oder in Kombination derselben;
Schritt 220: Videokeratoskop 20 aus dem Funktionszustand nehmen und in die Ausgangsposition zurückführen;
Schritt 221: Beurteilung und Entscheid ob die Topologie Erfassung der Cornea 18.3 ausreichend erfasst und abgeschlossen (beendet) ist oder ob das Videokeratoskop zur Analyse einer nächsten Cornea zu aktivieren ist, wenn
   a) JA (YES): Rückführung des Videokeratoskops 20 in den Be-triebsmodus gemäss Schritt 201 für die Einzelnen Funktionsabläufe 203 bis 220;
   b) NEIN (NO): weiter zu Schritt 222;
Schritt 222: Abschalten der gesamten Einrichtung 90.

Die Funktionstechnischen Merkmale und Schritte sowie die mechanischen und elektronischen Elemente sowie deren Anordnung zueinander sind nicht auf das vorstehend beschriebene Ausführungsbeispiel, insbesondere nicht auf das in Fig.1 und Fig.2 schematisch dargestellte Videokeratoskop 20 beschränkt.

## Patentansprüche

1. Einrichtung zum Ermitteln topographischer Eigenschaften der Cornea an einem Auge (18), mit einem zum Erfassen fotographischer Abbildungen ausgebildeten und mit einem Projektionskonus versehenen Videokeratoskop (20), einer daran angeordneten Trägereinheit (80) für eine hinsichtlich der Lage und Orientierung relativ zu der Cornea (18.3) elektromotorisch verschiebbare Positioniereinheit (85) mit daran angeordneter Indentationseinheit (35;135), welche eine in den Strahlengang (28") des Projektionskonus (28) ragende und zur Erreichung einer Indentation der Cornea (18.3) zuführbare Sonde (33;133) aufweist, **dadurch gekennzeichnet, dass** die Indentationseinheit (35;135) einen ersten Tragbügel (39;139) sowie einen unter Zwischenschaltung eines Elektromagneten (36;136) daran angeordneten und mit der Sonde (33;133) versehenen zweiten Tragbügel (34;134) umfasst, wobei der mit einstellbarem elektrischen Betriebsstrom kraftschlüssig an dem Elektromagneten (36;136) gehaltene zweite Tragbügel (34;134) beim Überschreiten einer vorbestimmten und elektronisch einstellbaren sowie überwachbaren Indentationskraft derart relativ zu dem Elektromagneten (36;136) bewegbar ist, dass die Sonde (33;133) zumindest mit der daran angeordneten Sondenspitze (32;132) ausser Eingriff der Vorderfläche der Cornea (18.3) bringbar ist.

2. Einrichtung nach Anspruch 1, **dadurch gekennzeichnet, dass** die Indentationseinheit (35;135) mit dem zweiten Tragbügel (34;134) und der daran angeordneten Sonde (33;133) für jede einzelne Indentation mit elektronisch einstellbaren sowie kraftabhängig und positionsabhängig vorbestimmten und elektronisch überwachten Daten sequentiell und linear der Vorderfläche der Cornea (18.3) zuführbar und nach erfolgter Indentation relativ zu der Vorderfläche der Cornea (18.3) in eine Ausgangsposition zurückführbar ist.

3. Einrichtung nach den Ansprüchen 1 und 2, **dadurch gekennzeichnet, dass** die Sonde (33;133) für die einzelne Indentation in Abhängigkeit einer Anzahl von dem mit einer Lichtquelle versehenen Videokeratoskop (20) auf die Vorderfläche der Cornea (18.3) projizierter Placido-Ringe (18.5) sequentiell und linear der Vorderfläche der Cornea (18.3) zuführbar ist.

4. Einrichtung nach einem der Ansprüche 1 bis 3, **dadurch gekennzeichnet, dass** die Sonde (33;133) nach dem Erfassen einer fotographischen Abbildung von der natürlichen Cornea mittels der vorbestimmten Daten der Cornea (18.3) für die Indentation zuführbar ist und nach dem Erfassen einer fotographischen Abbildung von der lokal bewirkten Verformung der Cornea (18.3) relativ zu der Vorderfläche der Cornea in die Ausgangsposition zurückführbar ist.

5. Einrichtung nach Anspruch 1, **dadurch gekennzeichnet, dass** der Elektromagnet (36) zwischen den beiden Tragbügeln (34,39) angeordnet und an der dem zweiten Tragbügel (34) zugewandten Seite zur Halterung desselben ein Zwischenstück (35') aus ferromagnetischem Material aufweist, mittels welchem der zweite Tragbügel (34) beim Überschreiten der eingestellten Indentationskraft relativ zu dem Elektromagneten (36) beziehungsweise zu dem Zwischenstück (35') kippbar und infolge davon die Sonde (33;133) mit der Sondenspitze (32;132) ausser Eingriff der Cornea (18.3) bringbar ist.

6. Einrichtung nach Anspruch 5, **dadurch gekennzeichnet, dass** der mit der Sonde (33) und dem Elektromagnet (36) versehene zweite Tragbügel (34) in Bezug auf die optische Achse (28') des Videokeratoskops (20) einstellbar und feststellbar an dem ersten Tragbügel (39) angeordnet ist.

7. Einrichtung nach Anspruch 1, **dadurch gekennzeichnet, dass** der Elektromagnet (136) mit dem einen Ende an dem ersten Tragbügel (139) angeordnet und der zweite Tragbügel (134) an zwei im Abstand zueinander an dem ersten Tragbügel (139) angeordnete und jeweils mit einem Kraftsensor (140,140') versehene Haltebolzen (192,142') derart gelagert ist, dass beim Überschreiten der vorbestimmten und eingestellten Indentationskraft der zweite Tragbügel (134) mittels der beiden Kraftsensoren (140,140') relativ zu dem Elektromagneten (136) verschiebbar und dabei die Sonde (133) mit der Sondenspitze (132) ausser Eingriff der Cornea (18.3) bringbar ist.

8. Einrichtung nach Anspruch 1, **dadurch gekennzeichnet, dass** der Projektionskonus (28) eine Ausnehmung (29) für die in den Strahlengang (28") ragende und hinsichtlich der Lage und Orientierung freibeweglich der Cornea (18.3) zuführbare und wieder entfernbare Sonde (33;133) aufweist.

9. Einrichtung nach einem der Ansprüche 1 bis 8, **dadurch gekennzeichnet, dass** die an der Sonde (33;133) angeordnete und mit der Vorderfläche der Cornea (18.3) in Eingriff bringbare Sondenspitze (32;132) für eine lokale Verformung, insbesondere für eine lokale konkave Verformung der Cornea (18.3) ausgebildet ist.

10. Einrichtung nach Anspruch 9, **dadurch gekennzeichnet, dass** die an der Sonde (33;133) angeordnete Sondenspitze (32;132) eine flache oder abgerundete beziehungsweise halbkugelförmig ausgebildete Stirnfläche aufweist.

11. Einrichtung nach Anspruch 1, **dadurch gekennzeichnet, dass** die Trägereinheit (80) mittels einer ersten Platte (40) sowie daran befestigter Stützen (41,41',45,45') an dem Videokeratoskop (20) angeordnet ist und zur Aufnahme und Lagerung der mit elektromotorischen Antrieben (62,63,64) und Stellgliedern (55,56,57) versehenen Positioniereinheit (85) eine zweite Platte (50) aufweist, welche derart an einem quer zu der optischen Achse (28') orientierten Bolzen (52) gelagert ist, dass die Indentationseinheit (35;135) mit der daran angeordneten Sonde (33;133) in Bezug auf die optische Achse (28') des Projektionskonus (28) einstellbar ist.

12. Einrichtung nach den Ansprüchen 1 und 2, **dadurch gekennzeichnet, dass** dem Videokeratoskop (20) eine Stützvorrichtung (15) mit daran angeordneten Kontaktsensoren (112,112',116) zugeordnet ist, mittels welcher die Sonde (33;133) zusätzlich zu den kraftabhängigen und positionsabhängigen Daten mit von dem Herzschlag des jeweiligen Patienten pulssynchron erfassten Daten der Cornea (18.3) zuführbar ist.

13. Einrichtung nach Anspruch 12, **dadurch gekennzeichnet, dass** die Stützvorrichtung (15) zwei Handgriffe (12,12') mit daran angeordneten ersten Kontaktsensoren (112,112') sowie eine Stirnstütze (16) mit einem daran angeordneten zweiten Kontaktsensor (116) aufweist.

14. Einrichtung nach einem der Ansprüche 1 bis 13, **gekennzeichnet durch** eine mit dem Videokeratoskop (20), der Stützvorrichtung (15), der Positioniereinheit (85) und der Indentationseinheit (35;135) in Wirkverbindung stehende Auswerteeinheit (95), welche eine Detektionseinheit (110), eine Steuereinheit (125) und einen Prozessor (150) umfasst, wobei
**a)** die Detektionseinheit (110) einen Oszillator (101), einen Verstärker (102), einen Gleichrichter (103), einen Frequenzfilter (104) sowie einen Vergleicher (105) aufweist und mit den Kontaktsensoren (112,112',116) der Stützvorrichtung (15) sowie mit der Indentationseinheit (35;135) unter Zwischenschaltung von Steuermitteln (145;145') elektrisch verbunden ist; und
**b)** dass die Steuereinheit (125) einen Regler (120) und einen Treiber (121) aufweist und mit den elektromotorischen Antrieben (62,63,64) der Positioniereinheit (85) elektrisch verbunden ist; und
**c)** dass der Prozessor (150) mit einer Erfassungseinheit (151) für die fotographischen Abbildungen sowie mit einer Regeleinheit (152) und einem Steuermodul (153) versehen ist und zudem mit einem Farbdrucker (154) und/oder einem Farbmonitor (155) elektrisch verbunden ist; und
**d)** dass der für die Indentationskraft der Sonde (33;133) an der Positioniereinheit (85) sowie der für den Elektromagneten (36;136) an der Indentationseinheit (35;135) anliegende elektrische Betriebsstrom jeweils von dem Prozessor (150) fix einstellbar beziehungsweise **durch** eine Vorwahl selbstregulierend einstellbar ist.

15. Einrichtung nach Anspruch 14, **dadurch gekennzeichnet, dass** die Detektionseinheit (110) unter Zwischenschaltung zusätzlicher Steuermittel (145') sowie mindestens eines Kraftsensors (140,140') mit dem Elektromagnet (136) und der damit wirkverbundenen Sonde (133) elektrisch verbunden ist.

16. Einrichtung nach den Ansprüchen 14 und 15, **dadurch gekennzeichnet, dass** der erforderliche elektrische Betriebsstrom für den Kraftsensor (140;140') und dem damit wirkverbundenen Elektromagnet (136) mittels des mit entsprechenden elektronischen Komponenten versehenen Prozessors (150) einstellbar ist.

## Claims

1. A device for determining the topographic properties of the cornea in an eye (18), with a video keratoscope (20) which is configured for detecting photographic images and provided with a projection cone, a support unit (80) arranged thereon for a positioning unit (85) displaceable in an electromotive manner relating to the position and orientation relative to the cornea (18.3) and comprising an indentation unit (35,135) which is arranged thereon and comprises a probe (33;133) projecting into the beam path (28") of the projection cone (28) and being advanced for achieving an indentation of the cornea (18.3), **characterized in that** the indentation unit (35;135) comprises a first support bracket (39;139) and a second support bracket (34;134) which is arranged thereon by interposing an electromagnet (36;136) and is provided with the probe (33;133), with the second support bracket (34;134) which is held with an adjustable electric operating current in a non-positive manner on the electromagnet (36;136), when exceeding a predetermined and electronically adjustable and supervisable indentation force, being movable relative to the electromagnet (36;136) that the probe (33;133) can be brought out of engagement with the front surface of the cornea (18.3) at least with the probe tip (32;132) which is arranged thereon.

2. A device according to claim 1, **characterized in that** the indentation unit (35;135) with the second support bracket (34;134) and the probe (33;133) arranged thereon can be advanced sequentially and linearly to the front surface of the cornea (18.3) for every single indentation with data which is electronically adjustable, predetermined depending on force and position and is monitored electronically, and can be returned to its initial position after complete indentation relative to the front surface of the cornea (18.3).

3. A device according to the claims 1 and 2, **characterized in that** the probe (33;133) can be advanced for the individual indentation sequentially and linearly to the front surface of the cornea (18.3) depending on a number of Placido rings (18.5) projected onto the front surface of the cornea (18.3) by the video keratoscope (20) provided with a light source.

4. A device according to one of the claims 1 to 3, **characterized in that** after taking a photographic image of the natural cornea the probe (33;133) can be advanced for the indentation by means of the predetermined data of the cornea (18.3) and, after taking a photographic image of the locally produced deformation of the cornea (18.3), can be returned to the initial position relative to the front surface of the cornea.

5. A device according to claim 1, **characterized in that** the electromagnet (36) is arranged between the two support brackets (34,39) and comprises an intermediate element (35') made of ferromagnetic material on the side facing the second support bracket (34) for fixing the same, by means of which the second support bracket (34) is tiltable upon exceeding the set indentation force relative to the electromagnet (36) or to the intermediate element (35') and as a result of the same the probe (33;133) can be brought out of engagement with the cornea (18.3) with the probe tip (32;132).

6. A device according to claim 5, **characterized in that** the second support bracket (34) which is provided with the probe (33) and the electromagnet (36) is adjustable with respect to the optical axis (28') of the video keratoscope (20) and is arranged in a fixable manner on the first support bracket (39).

7. A device according to claim 1, **characterized in that** the electromagnet (136) is arranged with the one end on the first support bracket (139) and the second support bracket (134) is held on two holding pins (142,142') which are arranged at a distance from each other on the first support bracket (139) and at each provided with a force sensor (140,140') in such a way that upon exceeding the predetermined and set indentation force the second support bracket (134) is displaceable by means of the two force sensors (140,140') relative to the electromagnet (136) and thereby the probe (133) can be brought out of engagement with the probe tip (132) of the cornea (18.3).

8. A device according to claim 1, **characterized in that** the projection cone (28) comprises a recess (29) for the probe (33;133) which projects into the beam path (28") and can be advanced and removed again in a freely movable way relative to the cornea (18.3) concerning position and orientation.

9. A device according to one of the claims 1 to 8, **characterized in that** the probe tip (32;132) which is arranged on the probe (33;133) and can be brought into engagement with the front surface of the cornea (18.3) is configured for a local deformation, especially for a local concave deformation of the cornea (18.3).

10. A device according to claim 9, **characterized in that** the probe tip (32;132) comprises a face surface which is configured in a flat or rounded manner or is provided with a semi-spherical configuration.

11. A device according to claim 1, **characterized in that** the support unit (80) is arranged by means of a first plate (40) and supports (41,41',45,45') fastened thereto on the video keratoscope (20) and comprises a second plate (50) for receiving and holding the positioning unit (85) which is provided with the electromotive drives (62,63,64) and actuators (55,56,57), which second plate is held on a pin (52) oriented transversally to the optical axis (28') so that the indentation unit (35;135) with the probe (33;133) arranged thereon is adjustable with respect to the optical axis (28') of the projection cone (28).

12. A device according to claim 1 and 2, **characterized in that** the video keratoscope (20) is associated with a support apparatus (15) with contact sensors (112,112',116) arranged thereon, with which the probe (33;133), in addition to the data dependent on force and position, can be advanced to the cornea (18.3) with the data obtained in a pulse-synchronous manner from the heartbeat of the respective patient.

13. A device according to claim 12, **characterized in that** the support apparatus (15) comprises two handles (12,12') with first contact sensors (112,112') arranged thereon and a forehead rest (16) with a second contact sensor (116) arranged thereon.

14. A device according to one of the claims 1 to 13, **characterised by** an evaluation unit (95) which is in operative connection with the video keratoscope (20), the support apparatus (15), the positioning unit (85) and the indentation unit (35;135) and which comprises a detection unit (110), a control unit (125) and a processor (150), wherein
**a)** the detection unit (110) comprising an oscillator (101), an amplifier (102), a rectifier (103), a frequency filter (104) as well as a comparator (105) and being electrically connected with the contact sensors (112,112',116) of the support apparatus (15) and with the indentation unit (35;135) by interposing control means (145;145'), and
**b)** that the control unit (125) comprises a controller (120) and a driver (121) and is electrically connected with the electromotive drives (62,63,64) of the positioning unit (85), and
**c)** that the processor (150) is provided with a frame grabbing unit (151) for the photographic images and with a controlling unit (152) and a control module (153) and is moreover connected with a color printer (154) and/or a color monitor (155), and
**d)** that the electric operating current applied for the indentation force of the probe (33;133) on the positioning unit (85) and for the electromagnet (36;136) on the indentation unit (35;135) can each be set in a fixed manner by the processor (150) or in a self-regulating manner by preselection.

15. A device according to claim 14, **characterized in that** the detection unit (110) is electrically connected with the electromagnet (136) and the probe (133) which is operatively connected with the same by interposing additional control means (145') and at least one force sensor (140,140').

16. A device according to claims 14 and 15, **characterized in that** the required electric operating current for the force sensor (140;140) and the electromagnet (136) which is operatively connected with the same, can be set by means of the processor (150) provided with the respective electronic components.

## Revendications

1. Dispositif destiné à déterminer les qualités topographiques de la cornée d'un oeil (18), comportant un vidéokératoscope (20) conçu pour capturer des images photographiques et équipé d'un cône de projection, une unité de support (80) disposée dessus pour une unité de positionnement (85) pouvant être déplacée par moteur électrique par rapport à la cornée (18.3) pour ce qui est de la position et de l'orientation, et comportant une unité d'indentation (35;135) disposée à son niveau, laquelle présente une sonde (33;133) dépassant dans le passage de faisceau (28") du cône de projection (28) et permettant d'atteindre la cornée (18.3) pour réaliser une indentation, **caractérisé en ce que** l'unité d'indentation (35;135) présente un premier étrier de support (39;139) et un second étrier de support (34;134), disposé à son niveau en intercalant un électroaimant (36;136) et équipé de la sonde (33;133), **en ce que** le second étrier de support (34;134), maintenu par adhérence au niveau de l'électroaimant (36;136) via un courant électrique de service réglable, lors d'un dépassement d'une force d'indentation prédéterminée et réglable et pouvant être surveillée par voie électronique, peut être déplacé par rapport à l'électroaimant (36;136) de façon telle que la sonde (33;133) au moins avec la pointe dé sonde (32;132) disposée à son niveau peut être mise hors de contact de la surface avant de la cornée (18.3).

2. Dispositif selon la revendication 1, **caractérisé en ce que** l'unité d'indentation (35;135), avec le second étrier de support (34;134) et la sonde fixée à son niveau (33;133), peut être amenée de façon séquentielle et linéaire, pour chaque indentation, sur la surface avant de la cornée (18.3), avec des données réglables par voie électronique, prédéterminées en fonction de la force et de la position, et pouvant être surveillées par voie électronique, et suite à une indentation réussie, peut être rétractée dans une position de départ par rapport à la surface avant de la cornée (18.3).

3. Dispositif selon la revendications 1 et 2, **caractérisé en ce que** la sonde (33;133), pour chaque indentation, peut être amenée de façon séquentielle et linéaire sur la surface avant de la cornée (18.3) en fonction d'un nombre de disques de Placido (18.5) projetés par le vidéokératoscope (20) équipé d'une source lumineuse sur la surface avant de la cornée (18.3).

4. Dispositif selon l'une des revendications 1 à 3, **caractérisé en ce que** la sonde (33;133), après la capture d'une image photographique de la cornée naturelle au moyen des données prédéfinies, peut être amenée pour l'indentation sur la cornée (18.3), et après capture d'une image photographique de la déformation provoquée localement de la cornée (18.3), peut être rétractée par rapport à la surface avant de la cornée, dans la position de départ.

5. Dispositif selon la revendication 1, **caractérisé en ce que** l'électroaimant (36) est disposé entre les deux étriers de suspension (34,39) et présente sur le côté orienté vers le second étrier de support (34), pour retenir celui-ci, un élément intermédiaire (35') réalisé dans un matériau ferromagnétique, au moyen duquel le second étrier de support (34), en cas de dépassement de la force d'indentation réglée, peut être basculé par rapport à l'électroaimant (36) ou par rapport à l'élément intermédiaire (35'), et la sonde (33;133) avec la pointe de sonde (32;132) peut alors être amenée hors de contact de la cornée (18.3).

6. Dispositif selon la revendication 5, **caractérisé en ce que** le second étrier de support (34), équipé de la sonde (33) et de l'électroaimant (36), est disposé au niveau du premier étrier de support (39) de façon pouvant être réglée et calée par rapport à l'axe optique (28') du vidéokératoscope (20).

7. Dispositif selon la revendication 1, **caractérisé en ce que** l'électroaimant (136) est disposé par une extrémité au niveau du premier étrier de support (139) et **en ce que** le second étrier de support (134) est logé au niveau de deux boulons d'arrêt (142,142') disposés en formant un écart entre eux au niveau du premier étrier de support (139) et munis respectivement d'un capteur de force (140,140'), de façon telle qu'en cas de dépassement de la force d'indentation prédéfinie et réglée, le second étrier de support (134) peut être déplacé au moyen des deux capteurs de force (140,140'), par rapport à l'électroaimant (136), et la sonde (133) avec la pointe de sonde (132) peut alors être mise hors du contact de la cornée (18.3).

8. Dispositif selon la revendication 1, **caractérisé en ce que** le cône de projection (28) présente un évidement (29) pour la sonde (33;133) qui dépasse dans le passage de faisceau (28") et peut être librement approchée et à nouveau éloignée de la cornée (18.3), pour ce qui est de sa position et de son orientation.

9. Dispositif selon l'une des revendications 1 à 8, **caractérisé en ce que** la pointe de sonde (32;132), disposée au niveau de la sonde (33;133) et pouvant être amenée en contact avec la surface avant de la cornée (18.3), est exécutée pour une déformation locale, en particulier pour une déformation concave locale de la cornée (18.3).

10. Dispositif selon la revendication 9, **caractérisé en ce que** la pointe de sonde (32;132) disposée au niveau de la sonde (33;133) présente une surface avant plane ou arrondie ou conçue en forme d'hémisphère.

11. Dispositif selon la revendication 1, **caractérisé en ce que** l'unité de support (80) est disposée, au moyen d'une première plaque (40) et d'appuis qui y sont fixés (41,41', 45,45'), sur le vidéokératoscope (20), et présente, pour placer et loger l'unité de positionnement (85) équipée d'entraînements (62,63,64) par moteur électrique et d'organes de réglage (55,56,57), une seconde plaque (50) qui est logée au niveau d'un boulon orienté transversalement à l'axe optique (28') de façon telle que l'unité d'indentation (35; 135) et la sonde qui lui est raccordée (33;133) peuvent être réglées par rapport à l'axe optique (28') du cône de projection (28).

12. Dispositif selon les revendications 1 et 2, **caractérisé en ce qu'**au vidéokératoscope (20) est coordonné un dispositif d'appui (15) avec des capteurs de contact (112,112',116) disposés à son niveau, au moyen desquels la sonde (33;133) peut être approchée de la cornée (18.3), en utilisant, en plus des données dépendant de la force et de la position, les données capturées par synchronisation de pulsation avec le battement cardiaque du patient respectif.

13. Dispositif selon la revendication 12, **caractérisé en ce que** le dispositif d'appui (15) comporte deux poignées (12,12') comportant des premiers capteurs de contact (112,112') disposés à leur niveau et un appuie-front (16) ayant un second capteur de contact (116) disposé à son niveau.

14. Dispositif selon l'une des revendications 1 à 13, **caractérisé par** une unité d'analyse (95), reliée par action coordonnée au vidéokératoscope (20), au dispositif d'appui (15), à l'unité de positionnement (85) et à l'unité d'indentation (35;135), laquelle comprend une unité de détection (110), une unité de commande (125) et un processeur (150), où
**a)** l'unité de détection (110) comprend un oscillateur (101), un amplificateur (102), un redresseur de courant (103), un filtre de fréquence (104) et un comparateur (105) et est reliée électriquement avec les capteurs de contact (112,112',116) de l'unité d'appui (15) et avec l'unité d'indentation (35;135), en intercalant des moyens de commande (145;145'); et
**b)** l'unité de commande (125) comprend un régulateur (120) et un circuit d'attaque (121) et est reliée électriquement aux entraînements (62,63,64) par moteur électrique de l'unité de positionnement (85); et
**c)** le processeur (150) est équipé d'une unité de capture (151) pour les images photographiques, et est muni d'une unité de régulation (152) et d'un module de commande (153), et est de plus relié électriquement à une imprimante couleur (154) et/ou à un moniteur couleur (155); et
**d)** le courant électrique de service appliqué sur l'unité de positionnement (85) pour la force d'indentation de la sonde (33;133), et sur l'unité d'indentation (35;135) pour l'électroaimant (36;136) peut être réglé respectivement de façon fixe par le processeur (150) ou selon le cas de façon auto-régulante par une présélection.

15. Dispositif selon la revendication 14, **caractérisé en ce que** l'unité de détection (110) est reliée électriquement à l'électroaimant (136) et à la sonde qui lui est reliée par action coordonnée, en intercalant des moyens de commande supplémentaires (145') et au moins un capteur de force (140,140').

16. Dispositif selon les revendications 14 et 15, **caractérisé en ce que** le courant électrique de service nécessaire pour le capteur de force (140,140') et l'électroaimant (136) relié par action coordonnée à celui-ci peut être réglé au moyen du processeur (150) muni de composants électroniques correspondants.
